# FASCICULE DE BREVET EUROPEEN

(11) **EP 1 771 492 B1**
(45) Date de publication et mention de la délivrance du brevet: **10.11.2010**
(21) Numéro de dépôt: 05778630.3
(22) Date de dépôt: 21.06.2005
(51) Int. Cl.: C08G 61/08

(54) **PARTICULES POLYMERES STIMULABLES PRESENTANT DES FONCTIONS REACTIVES, LEUR PROCEDE D'OBTENTION ET LEURS UTILISATIONS**
STIMULIERBARE POLYMERPARTIKEL MIT REAKTIVEN FUNKTIONEN, VERFAHREN ZUR HERSTELLUNG DAFÜR UND VERWENDUNG DAMIT
STIMULATEABLE POLYMER PARTICLES EXHIBITING REACTIVE FUNCTIONS, METHOD FOR THE PRODUCTION AND THE USE THEREOF

(30) Priorité: 21.06.2004 FR 0406707
(43) Date de publication de la demande: 11.04.2007
(73) Titulaire: CENTRE NATIONAL DE LA RECHERCHE SCIENTIFIQUE, 75794 Paris Cedex 16 (FR); INSTITUT NATIONAL DE LA SANTE ET DE LA RECHERCHE MEDICALE (INSERM), 75654 Paris Cédex 13 (FR); UNIVERSITE DE BORDEAUX I, 33405 Talence Cedex (FR); Ecole Nationale Superieure de Chimie Et de Physique de Bordeaux 1, 33607 Pessac Cedex (FR)
(72) Inventeur: SABAUT-HEROGUEZ, Valérie, F-33700 Merignac (FR); QUEMENER, Damien, F-54000 NANCY (FR); DURRIEU, Marie-Christine, F-33140 Villenave d'Ornon (FR)
(74) Mandataire: Domenego, Bertrand
(86) Numéro de dépôt international: PCT/FR2005/001546
(87) Numéro de publication internationale: WO 2006/008387

(56) Documents cités:
- DELAUDE L ET AL: "HIGHLY STEREOSELECTIVE RUTHENIUM-CATALYZED RING-OPENING METATHESIS POLYMERIZATION OF 2,3-DIFUNCTIONALIZED NORBORNADIENES AND THEIR 7-OCA ANALOGUES" MACROMOLECULES, AMERICAN CHEMICAL SOCIETY. EASTON, US, vol. 32, no. 7, 6 avril 1999 (1999-04-06), pages 2091-2103, XP000823809 ISSN: 0024-9297
- DELAUDE L ET AL: "RUTHENIUM-BASED CATALYSTS FOR THE RING-OPENING METATHESIS POLYMERIZATION (ROMP) OF FUNCTIONALIZED CYCLIC OLEFINS" MACROMOLECULAR SYMPOSIA, WILEY VCH, WEINHEIM, DE, no. 153, mars 2000 (2000-03), pages 133-144, XP000991932 ISSN: 1022-1360

## Description

La présente invention a pour objet des particules polymères, et plus particulièrement des nanoparticules polymères, stimulables, c'est-à-dire sensibles à un stimuli extérieur tel qu'une variation de pH ou de température, présentant des fonctions réactives, notamment de type acide, amine, alcool, ou chlorure d'acide, en périphérie, ainsi que leur procédé de synthèse en une étape, et leurs utilisations.

Des polymères stimulables présentant des fonctions réactives obtenues par encapsulation ou adsorption des principes actifs directement dans le matériau ou dans des billes elles mêmes adsorbées ou greffées sur le matériau, ont déjà été décrits.

Toutefois, l'adsorption ne permet pas une libération contrôlée du principe actif. Quant à l'encapsulation, si elle peut permettre une libération contrôlée du principe actif, elle est en revanche incompatible avec une utilisation prolongée et/ou lorsque le matériau est soumis à de fortes contraintes (flux, frottement...).

Des nanoparticules polymères réactives obtenues par greffage covalent de principes actifs sur la nanoparticule fonctionnalisée ont également déjà été décrites. Cependant, la synthèse de telles nanoparticules se fait en deux étapes (synthèse du latex puis réaction avec le principe actif) et donc sans contrôle direct du greffage (nombre de fonctions introduites aléatoire). Par ailleurs ces nanoparticules ne possèdent pas conjointement le principe actif et des sites d'ancrages permettant une libération en un endroit spécifique du principe actif. Ces matériaux sont le plus souvent destinés à la vectorisation ou aux tests immunologiques.

La présente invention a pour but de fournir de nouvelles particules polymères, comportant une fonction réactive, le cas échéant engagée dans une liaison avec un principe actif, ou une molécule biologique telle qu'une protéine, ladite fonction réactive étant liée de façon covalente auxdits polymères, ces derniers étant obtenus en une seule étape.

L'invention concerne des particules sphériques d'un diamètre compris entre 10 nm et 100 µm, lesdites particules étant constituées par des chaînes polymère contenant environ 30 à 10000 unités monomères, identiques ou différentes, dérivées de la polymérisation d'alcènes monocycliques dont le nombre d'atomes de carbone constitutifs du cycle est d'environ 4 à 12, ou d'alcènes polycycliques dont le nombre total d'atomes de carbone constitutifs des cycles est d'environ 6 à 20, l'une au moins desdites unités monomères étant substituée par une chaîne R comprenant un polyoxyde d'éthylène de formule (A) le cas échéant lié de façon covalente auxdites unités monomères via un pont hydrolysable

-(CH₂-CH₂-O)ₙ-X (A)

dans laquelle n représente un nombre entier d'environ 50 à 340, notamment de 70 à 200, et X représente une chaîne alkyle ou alkyloxy d'environ 1 à 10 atomes de carbone, comprenant une fonction réactive du type OH, halogène, NH₂, C(O)X₁ dans laquelle X₁ représente un atome d'hydrogène, d'halogène, un groupe OR' ou NHR' dans lequel R' représente un atome d'hydrogène ou une chaîne hydrocarbonée d'environ 1 à 10 atomes de carbone, substituée ou non, ou X représente un groupe comprenant une fonction photosensible telle que les polyènes, ladite fonction réactive étant le cas échéant engagée dans une liaison avec un principe actif, ou une molécule biologique telle qu'une protéine, ladite chaîne R étant liée de façon covalente audit monomère.

L'invention a plus particulièrement pour objet des particules sphériques telles que définies ci-dessus, caractérisées en ce que les unités monomères sont dérivées de la polymérisation d'alcènes monocycliques, et sont de formule (Z1) suivante

=[CH-R₁-CH]= (Z1)

dans laquelle R₁ représente une chaîne hydrocarbonée de 2 à 10 atomes de carbone, saturée ou non, lesdites unités monomères étant le cas échéant substituées par une chaîne R, ou directement par un groupe X, tels que définis ci-dessus.

L'invention concerne plus particulièrement des particules sphériques telles que définies ci-dessus, caractérisées en ce que les alcènes monocycliques dont sont issues les unités monomères sont :
- le cyclobutène conduisant à un polymère comprenant des unités monomères de formule (Z1a) suivante : 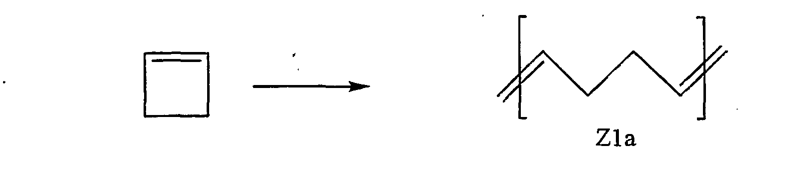
- le cyclopentène conduisant à un polymère comprenant des unités monomères de formule (Z1b) suivante : 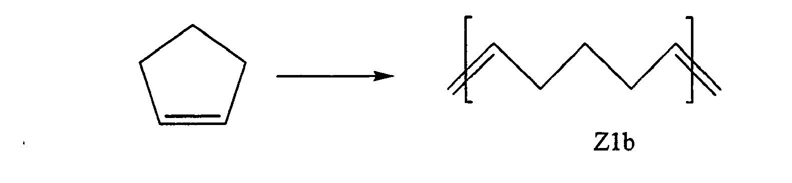
- le cyclopentadiène conduisant à un polymère comprenant des unités monomères de formule (Z1c) suivante 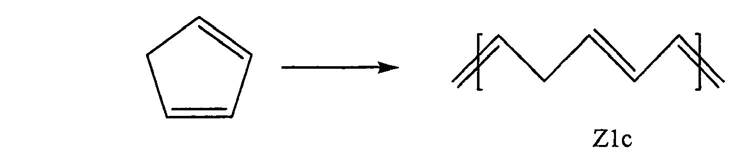
- le cyclohexène conduisant à un polymère comprenant des unités monomères de formule (Z1d) suivante 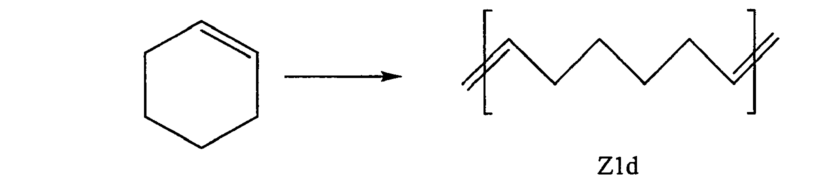
- le cyclohexadiène conduisant à un polymère comprenant des unités monomères de formule (Z1e) suivante 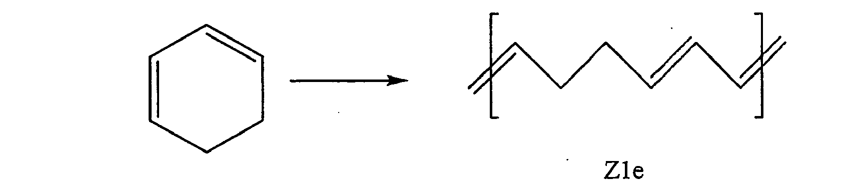
- le cycloheptène conduisant à un polymère comprenant des unités monomères de formule (Z1f suivante 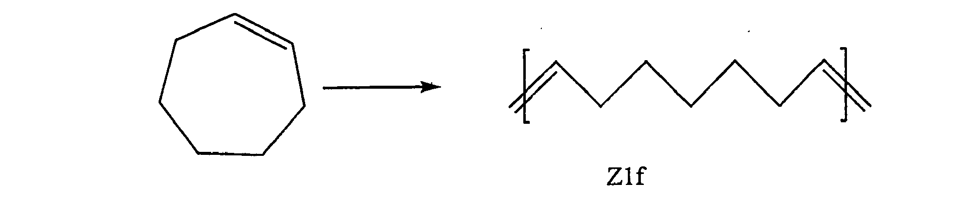
- le cyclooctène conduisant à un polymère comprenant des unités monomères de formule (Z1h) suivante 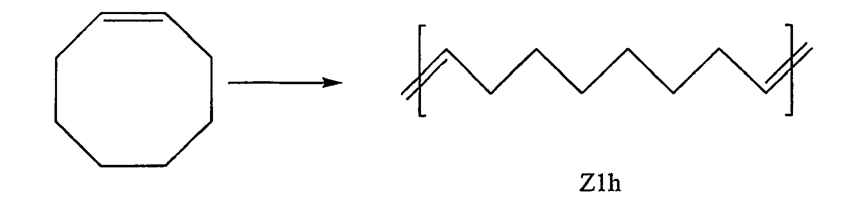
- le cyclooctapolyène, notamment le cycloocta-1,5-diene conduisant à un polymère comprenant des unités monomères de formule (Z1i) suivante 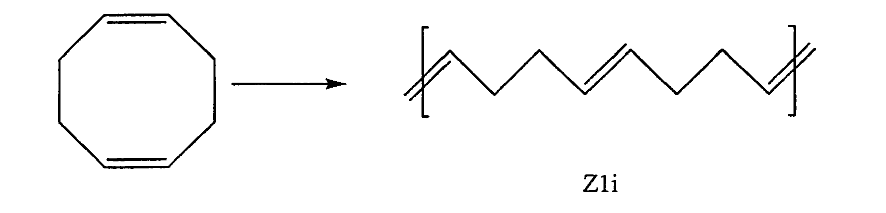
- le cyclononène conduisant à un polymère comprenant des unités monomères de formule (Z1j) suivante 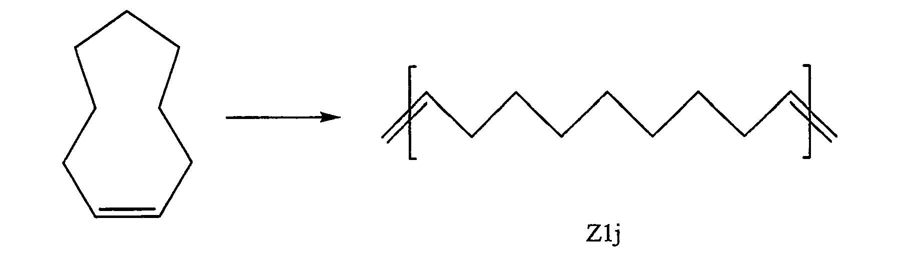
- le cyclononadiène conduisant à un polymère comprenant des unités monomères de formule (Z1k) suivante 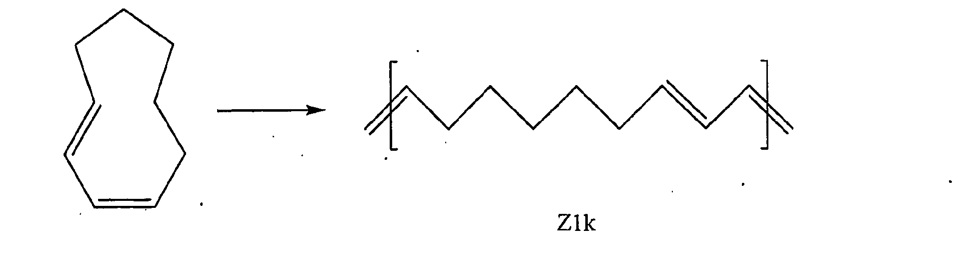
- le cyclodécène conduisant à un polymère comprenant des unités monomères de formule (Z1l) suivante 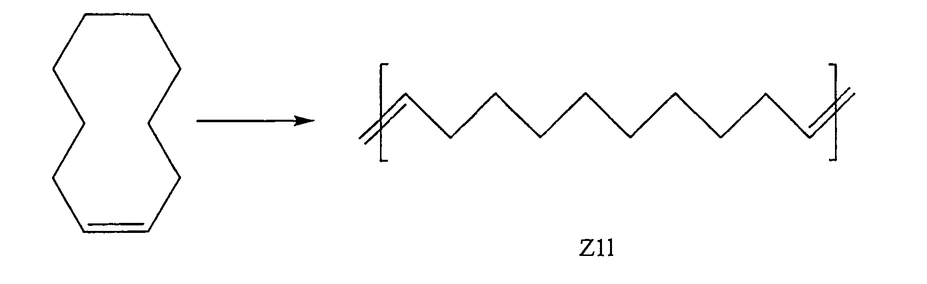
- le cyclodéca-1,5-diène conduisant à un polymère comprenant des unités monomères de formule (Z1m) suivante 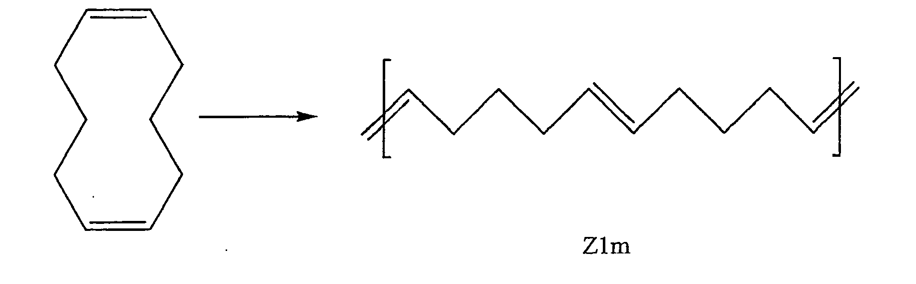
- le cyclododécène conduisant à un polymère comprenant des unités monomères de formule (Z1n) suivante 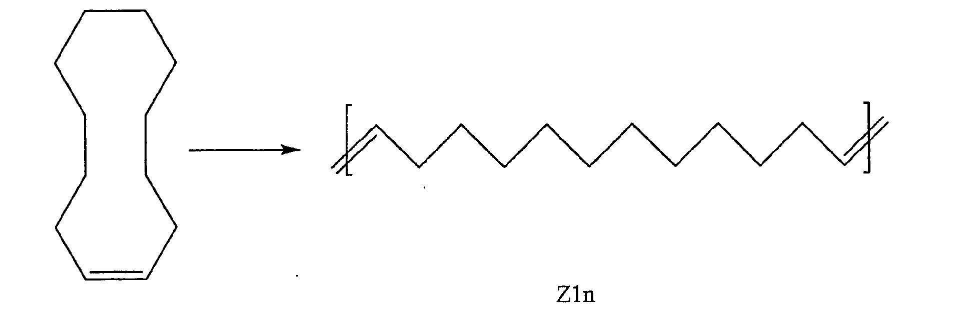
- ou encore l'acétate du 2,3,4,5-tétrahydrooxepin-2-yl, le cyclopentadécène, le paracyclophane, le ferrocenophane.

L'invention concerne également des particules sphériques telles que définies ci-dessus, caractérisées en ce que les unités monomères sont dérivées de la polymérisation d'alcènes polycycliques, et sont :
- de formule (Z2) suivante

   =[CH-R₂-CH]= (Z2)

   dans laquelle R₂ représente :
   * un cycle de formule 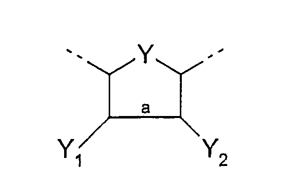 dans laquelle :
      - Y représente -CH₂-, ou un hétéroatome, ou un groupe -CHR-, ou un groupe -CHX-, R et X étant tels que définis ci-dessus,
      - Y1 et Y2, indépendamment l'un de l'autre, représentent H, ou une chaîne R, ou un groupe X susmentionnés, ou forment en association avec les atomes de carbone qui les portent un cycle de 4 à 8 atomes de carbone, ce cycle étant le cas échéant substitué par une chaîne R, ou un groupe X susmentionnés,
      - a représente une simple ou double liaison,
   * ou un cycle de formule 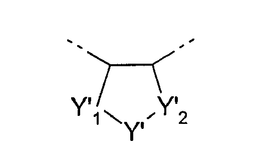 dans laquelle :
      - Y' représente -CH₂-, ou un hétéroatome, ou un groupe -CHR-, ou un groupe -CHX-, R et X étant tels que définis ci-dessus,
      - Y'1 et Y'2, indépendamment l'un de l'autre, représentent -CH₂-, ou un groupe -C(O), ou un groupe -COR, ou un groupe -C-OX, R et X étant tels que définis ci-dessus,
- de formule (Z3) suivante 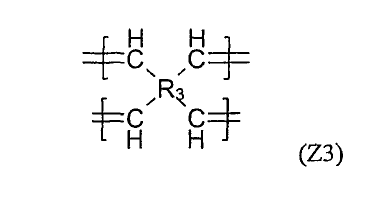 dans laquelle R₃ représente :
   * un cycle de formule 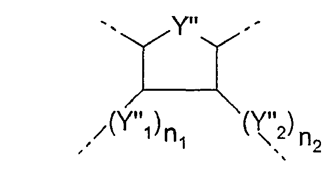 dans laquelle :
      . n1 et n2, indépendamment l'un de l'autre, représentent 0 ou 1,
      . Y" représente -CH₂-, ou un groupe -CHR-, ou un groupe -CHX-, R et X étant tels que définis ci-dessus,
      . Y"1 et Y"2, indépendamment l'un de l'autre, représentent une chaîne hydrocarbonée de 0 à 10 atomes de carbone,
   * ou un cycle de formule 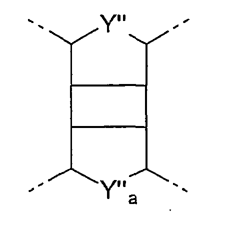 dans laquelle Y" et Y"a, indépendamment l'un de l'autre, représentent -CH₂-, ou un groupe -CHR-, ou un groupe -CHX-, R et X étant tels que définis ci-dessus.
      - ou un cycle de formule 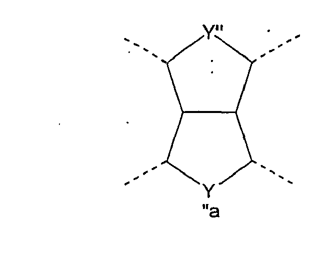
dans laquelle Y" et Y"a, indépendamment l'un de l'autre, représentent -CH₂-, ou un groupe -CHR-, ou un groupe -CHX-, R et X étant tels que définis ci-dessus.

L'invention a plus particulièrement pour objet des particules sphériques telles que définies ci-dessus, caractérisées en ce que les alcènes polycycliques dont sont issues les unités monomères sont :
- les monomères contenant un cycle cyclobutène conduisant à un polymère comprenant des unités monomères de formule (Z2a) suivante : 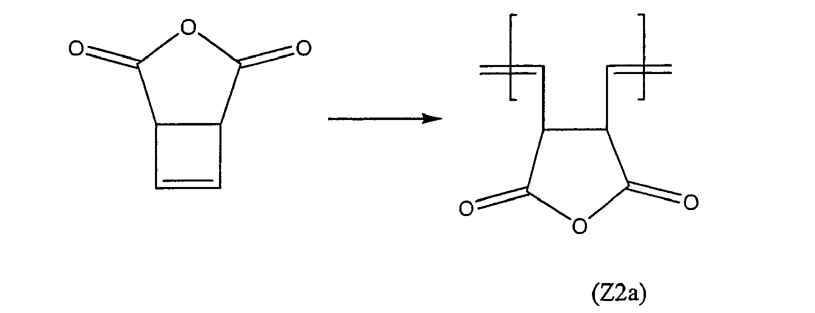
- les monomères contenant un cycle cyclopentène conduisant à un polymère comprenant des unités monomères de formule (Z2b) suivante : 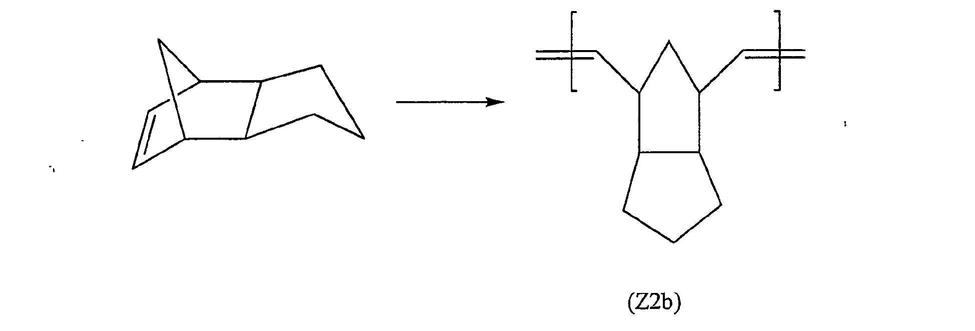
- le norbornène (bicyclo[2.2.1]hept-2-ène) conduisant à un polymère comprenant des unités monomères de formule (Z2c) suivante : 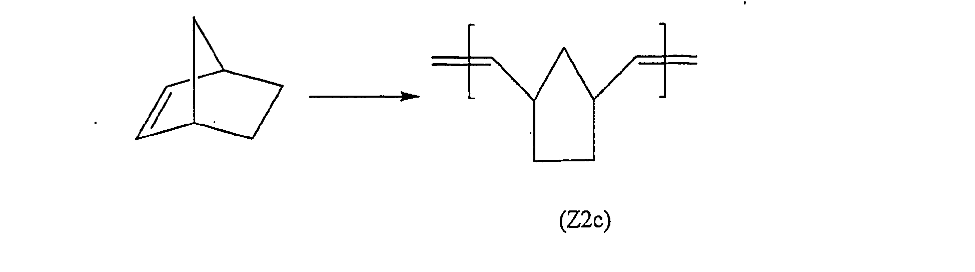
- le norbornadiène conduisant à un polymère comprenant des unités monomères de formule (Z2d) suivante : 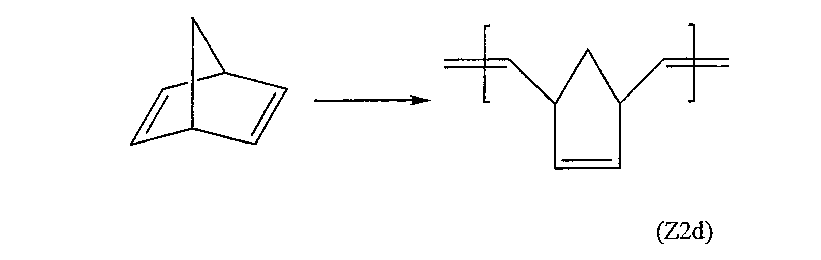
- le 7-oxanorbornène conduisant à un polymère comprenant des unités monomères de formule (Z2e) suivante : 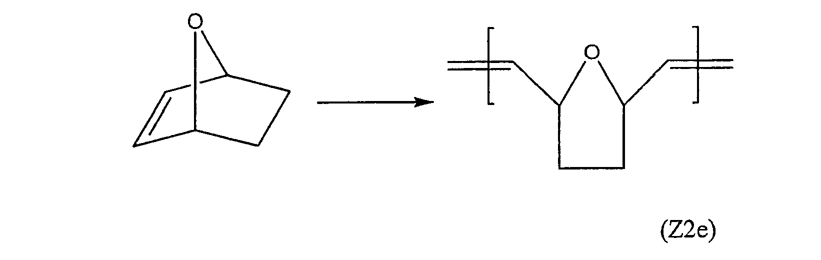
- le 7-oxanorbornadiène conduisant à un polymère comprenant des unités monomères de formule (Z2f) suivante : 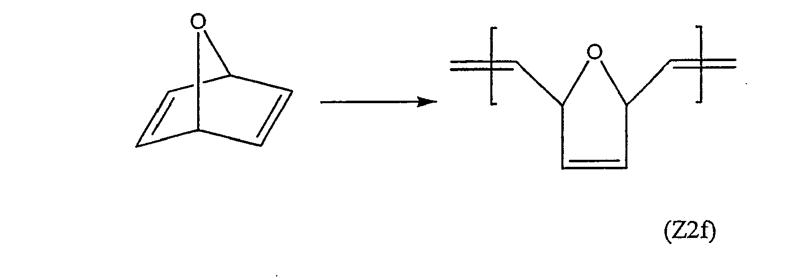
- le dimère du norbornadiène conduisant à un polymère comprenant des unités monomères de formule (Z3a) suivante : 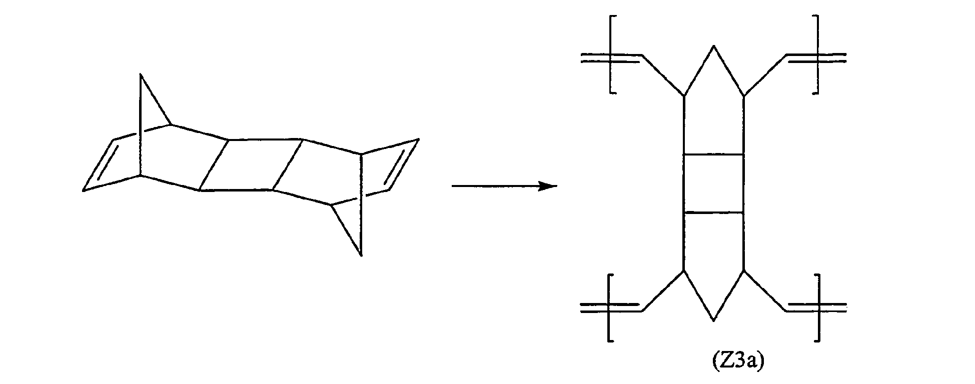
- le dicyclopentadiène conduisant à un polymère comprenant des unités monomères de formule (Z3b) suivante : 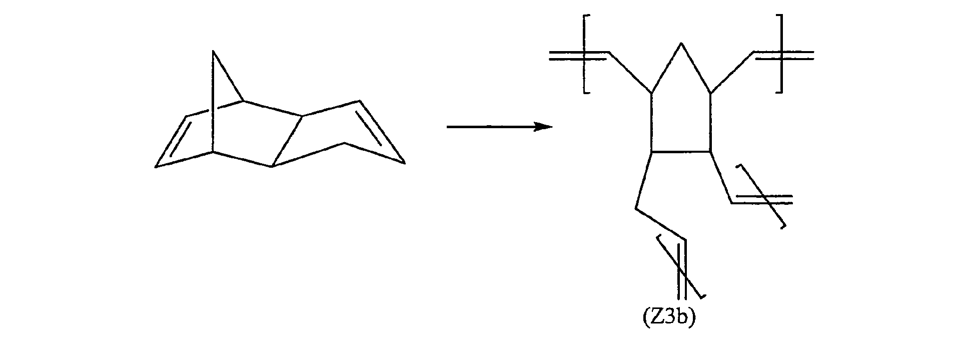
- le tetracyclododécadiene conduisant à un polymère comprenant des unités monomères de formule (Z3c) suivante : 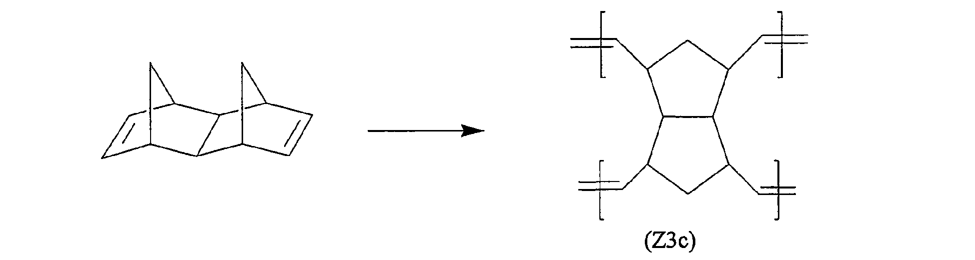
- ou le bicyclo[5.1.0]oct-2-ène, le bicyclo[6.1.0]non-4-ène.

L'invention concerne plus particulièrement des particules sphériques préférées telles que définies ci-dessus, caractérisées en ce que les alcènes mono ou polycycliques dont sont issues les unités monomères sont :
- le norbornène (bicyclo[2.2.1]hept-2-ène) conduisant à un polymère comprenant des unités monomères de formule (Z2c),
- le tetracyclododécadiene conduisant à un polymère comprenant des unités monomères de formule (Z3c),
- le dicyclopentadiène conduisant à un polymère comprenant des unités monomères de formule (Z3b),
- le dimère du norbornadiène conduisant à un polymère comprenant des unités monomères de formule (Z3a),
- le cycloocta-1,5-diene conduisant à un polymère comprenant des unités monomères de formule (Zli).

Avantageusement, les particules sphériques telles que définies ci-dessus, sont caractérisées en ce que au moins 0,5 % jusqu'à 100% des unités monomères sont substituées par une chaîne R telle que définie ci-dessus.

L'invention a plus particulièrement pour objet des particules sphériques telles que définies ci-dessus, caractérisées en ce qu'elles comprennent :
- entre environ 0,5 % et 99,5 % d'unités monomères substituées par une chaîne R telle que définie ci-dessus, ladite chaîne R étant identique pour ces monomères,
- et entre environ 0,5 % et 99,5 % d'unités monomères substituées par une chaîne R telle que définie ci-dessus, ladite chaîne R de ces monomères étant différente de la chaîne R des monomères précédents,
- et/ou entre environ 0,5 % et 99,5 % d'unités monomères substituées directement par un groupe X tel que défini ci-dessus, ce groupe X de ces monomères étant identique ou différent du groupe X de la chaîne R des monomères précédents,
- et/ou entre environ 0,5 % et 99,5 % d'unités monomères non substituées,
le total des pourcentages des différents monomères susmentionnés faisant 100 %.

L'invention concerne plus particulièrement des particules sphériques telles que définies ci-dessus, caractérisées en ce que la ou les chaînes R substituant les monomères sont représentées par la formule

-CH₂-O-(CH₂-CH₂-O)ₙ-CH₂-CH₂-O-X

dans laquelle n est tel que défini ci-dessus, et X représente H, -CH₂-COOH, -CH₂-COCl, -CH₂-COY, Y représentant un principe actif, ou une molécule biologique telle qu'une protéine.

L'invention concerne également des particules sphériques telles que définies ci-dessus caractérisées en ce que la ou les chaînes R comprennent un polyoxyde d'éthylène de formule (A) lié de façon covalente auxdites unités monomères via un pont hydrolysable.

De telles particules sphériques sont particulièrement avantageuses dans la mesure où elles permettent une libération contrôlée de principes actifs stables ou pas in vivo. Suivant cette stratégie la libération du principe actif, emprisonné à l'intérieur de la particule, donc isolé du milieu extérieur, et lié de manière covalente à celle-ci, se fait par une première étape de déstabilisation desdites particules par rupture des liaisons entre les unités monomères et les chaînes R via un stimuli extérieur (tel que pH, hyperthermie...), ce qui entraîne un relargage des chaînes R stabilisantes. Les chaînes résultantes, R ou Z1, fonctionnalisées ou non par le principe actif, subissent dans un deuxième temps des réactions d'hydrolyses et libèrent le principe actif.

Les particules sphériques de l'invention sont donc des particules stimulables, c'est-à-dire sensibles à un stimuli extérieur tel qu'une variation de pH ou de température, ce qui permet alors la libération des principes actifs emprisonnés à l'intérieur de ces particules.

De préférence les ponts hydrolysables susmentionnés sont choisis parmi les enchaînements d'environ 1 à 10 motifs d'ε-caprolactone, ou de fonctions -OC(O)-, -C(O)OC(O)-, -C(O)-NH-... A ce titre, l'invention a plus particulièrement pour objet des particules sphériques telles que définies ci-dessus, caractérisées en ce que la ou les chaînes R comprenant un polyoxyde d'éthylène de formule (A) lié de façon covalente à un pont hydrolysable choisi parmi les enchaînements d'environ 1 à 10 motifs d'ε-caprolactone, sont représentées par la formule

-CH₂-(O-CO-(CH₂)₅)ₜ-O-CO-(CH₂)₅-O-CO-(CH₂)₂-CO-O-(CH₂-CH₂-O)ₙ-(CH₂)₂-O-X

dans laquelle t représente un nombre entier compris entre 1 et 10, et X représente H, -CH₂-COOH, -CH₂-COCl, ou -CH₂-COY, Y représentant un principe actif, ou une molécule biologique telle qu'une protéine.

L'invention concerne également des particules sphériques telles que définies ci-dessus, caractérisées en ce que le principe actif est choisi parmi les molécules utilisées en thérapeutique, cosmétique, parfumerie, ou pour les revêtements de surface, tels que les peintures et les antifoulings.

L'invention a plus particulièrement pour objet des particules sphériques telles que définies ci-dessus, caractérisées en ce que le principe actif est un médicament utilisé en thérapeutique choisi notamment parmi ceux des classes thérapeutiques suivantes : les anti-inflammatoires, notamment l'indométacine, les anti-cancéreux, les antibiotiques, les anti-coagulants, ou les antimitotiques.

L'invention concerne plus particulièrement des particules sphériques telles que définies ci-dessus, caractérisées en ce que la molécule biologique est choisie parmi les protéines susceptibles de se lier à une cible biologique intra ou extracellulaire, ou à des anticorps, ou à tout autre ligand spécifique.

L'invention a plus particulièrement pour objet des particules sphériques telles que définies ci-dessus, caractérisées en ce que la molécule biologique est choisie parmi les protéines suivantes : l'avidine, l'albumine, les facteurs de croissance tel que le VEGF.

L'invention concerne également des compositions pharmaceutiques comprenant des particules sphériques telles que définies ci-dessus, dans lesquelles le ou les différents groupes X contiennent un principe actif médicamenteux, le cas échéant en association avec un véhicule pharmaceutiquement acceptable, notamment pour une utilisation sous forme parentérale.

L'invention a également pour objet des compositions cosmétiques comprenant des particules sphériques telles que définies ci-dessus, dans lesquelles le ou les différents groupes X contiennent un principe actif utilisé en cosmétique, le cas échéant en association avec un véhicule approprié, notamment pour une application sous forme d'émulsions, crèmes.

L'invention concerne également des compositions de revêtement de surfaces comprenant des particules sphériques telles que définies ci-dessus, dans lesquelles le ou les différents groupes X contiennent un principe actif utilisé pour les revêtements de surfaces, le cas échéant en association avec un véhicule approprié.

L'invention a également pour objet un procédé de préparation de particules sphériques telles que définies ci-dessus, caractérisé en ce qu'il comprend une étape de polymérisation d'un alcène mono ou polycyclique tel que défini ci-dessus substitué par une chaîne R telle que définie ci-dessus, le cas échéant en présence :
- d'un ou plusieurs alcènes mono ou polycycliques tels que définis ci-dessus, identiques ou différents du précédent, et substitués par une chaîne R telle que définie ci-dessus, ladite chaîne R étant différente de celle substituant l'alcène mono ou polycyclique susmentionné,
- et/ou d'un ou plusieurs alcènes mono ou polycycliques tels que définis ci-dessus, identiques ou différents du précédent, et substitués par un groupe X tel que défini ci-dessus, ce groupe X étant identique ou différent du groupe X de la chaîne R des alcènes précédents,
- et/ou d'un ou plusieurs alcènes mono ou polycycliques tels que définis ci-dessus, identiques ou différents des précédents, lesdits alcènes n'étant pas substitués,
ladite polymérisation étant effectuée sous agitation en présence d'un complexe de métal de transition en tant qu'amorceur de la réaction choisi notamment parmi ceux des groupes IV ou VI ou VIII, tel que le ruthénium, l'osmium, le molybdène, le tungstène, l'iridium, le titane, en milieu polaire ou apolaire, notamment à l'aide des complexes à base de ruthénium suivants : RuCl₃, RuCl₂(PCy₃)₂CHPh.

L'invention concerne également des alcènes mono ou polycycliques, caractérisés en ce qu'ils sont substitués par une chaîne R, ou un groupe X, tels que définis ci-dessus, ledit alcène de formule suivante étant exclu : 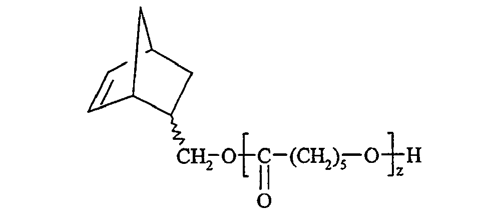 dans laquelle z représente un nombre entier compris entre 1 et 340.

Des alcènes mono ou polycycliques tels que définis ci-dessus préférés sont choisis parmi ceux mentionnés ci-dessus.

L'invention a plus particulièrement pour objet des alcènes mono ou polycycliques tels que définis ci-dessus, caractérisés par les formules suivantes : 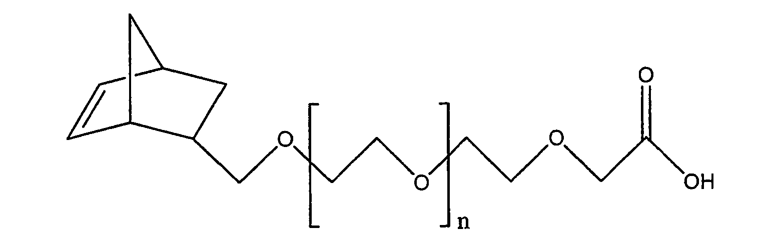 dans laquelle n représente un nombre entier compris entre environ 50 et 340, 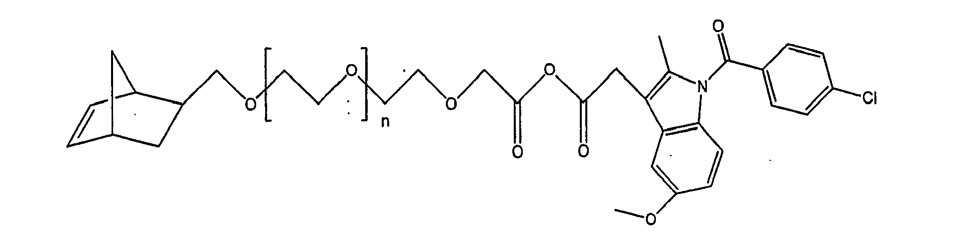 dans laquelle n représente un nombre entier compris entre environ 50 et 340, 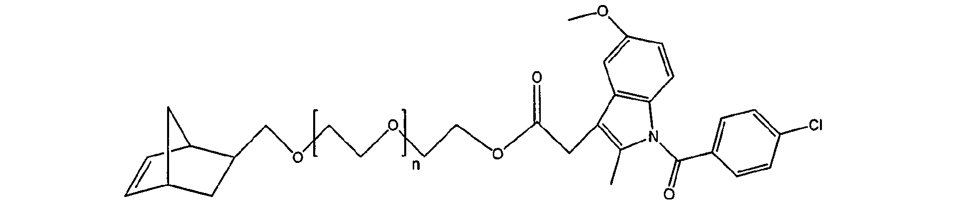 dans laquelle n représente un nombre entier compris entre environ 50 et 340, 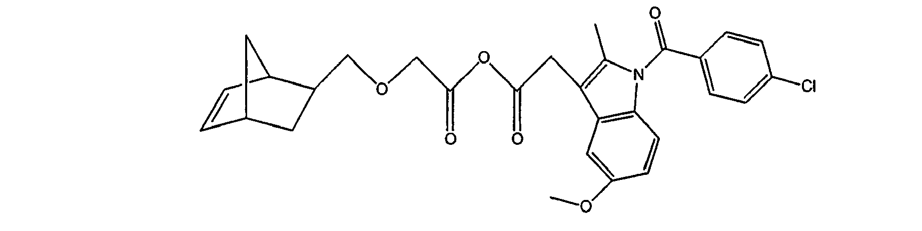 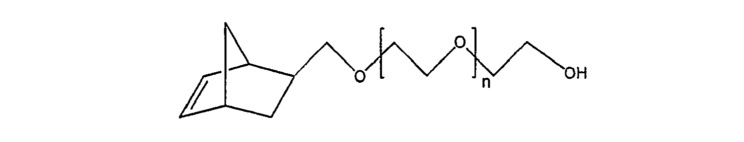 dans laquelle n représente un nombre entier compris entre environ 50 et 340, 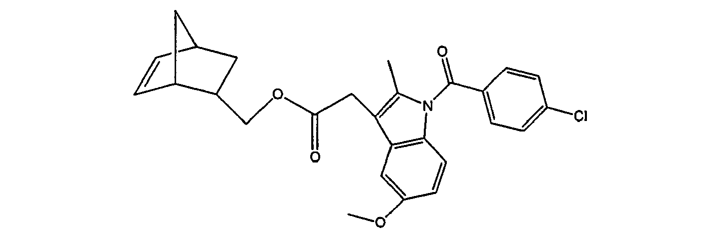 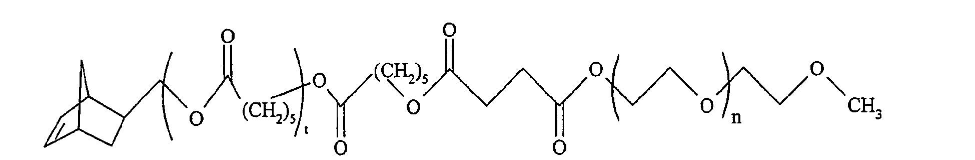 dans laquelle t représente un nombre entier compris entre 1 et 10, et n représente un nombre entier compris entre environ 50 et 340.

L'invention a également pour objet l'utilisation d'alcènes mono ou polycycliques tels que définis ci-dessus, pour la mise en oeuvre d'un procédé de préparation de particules sphériques définies ci-dessus, notamment selon le procédé décrit ci-dessus.

L'invention sera davantage illustrée à l'aide de la description détaillée qui suit de l'obtention de particules sphériques de l'invention, et de leurs caractéristiques physicochimiques.

### A) synthèse des macromonomères de formules A et B ci-après

Les macromonomères (A et B) sont des oligomères de poly(oxyde d'éthylène) de 7000 g/mol de masse molaire (M̅n). Ils sont issus d'une polymérisation anionique 'vivante' ce qui permet de contrôler la longueur et la fonctionnalité des chaînes. Ils sont fonctionnalisés à l'une de leur extrémité par une unité norbornényle, entité choisie pour sa réactivité élevée en polymérisation par métathèse et, à l'autre extrémité par une fonction réactive de type alcool, acide, amine...(A), ou par le principe actif (indométacine) (B) via un pont clivable (anhydride d'acide, ester, amide, ...).

### 1. α-norbornenyl-ω-carboxylic acid-poly(ethylene oxide) ; formule A

### Formule chimique :

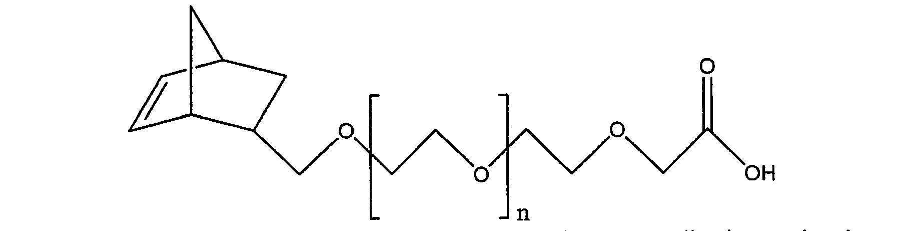

avec n compris entre 50 et 340 en fonction des besoins de l'application envisagée.

Référence : NB-POE-COOH.

### Schéma de synthèse:

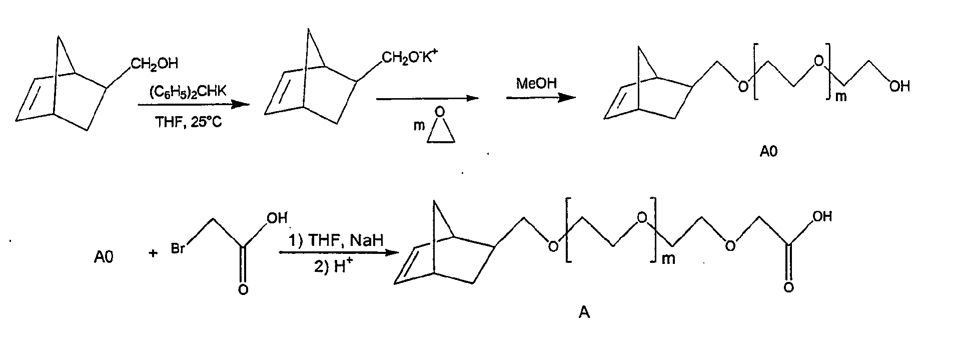

### Procédure de synthèse :

Le 5-norbomène-2-méthanol (0,5 mL) en solution dans le tétrahydrofurane (THF) (200mL) est tout d'abord déprotonné par l'ajout d'un équivalent molaire de diphénylméthyl potassium. L'espèce résultante va ensuite amorcer la polymérisation de l'oxyde d'éthylène (28 mL) de manière « vivante » (48h) jusqu'à la destruction des centres actifs par l'ajout de méthanol (1mL). La fonction alcool du poly(oxyde d'éthylène) obtenu (A0) va alors être transformée en fonction acide par déprotonnation de A0 ( 10g) avec NaH (0,17 g) en solution dans le THF (15mL), suivi de l'ajout d'acide bromoacétique (0,42g). Après lavage du produit à l'acide chlorhydrique (18 mL, 1M) puis précipitation dans l'éther, le macromonomère A est obtenu sous une forme pure.

### 2. α-norbornenyl-ω-indométacine-poly(ethylene oxide) ; formule B

### Formule chimique :

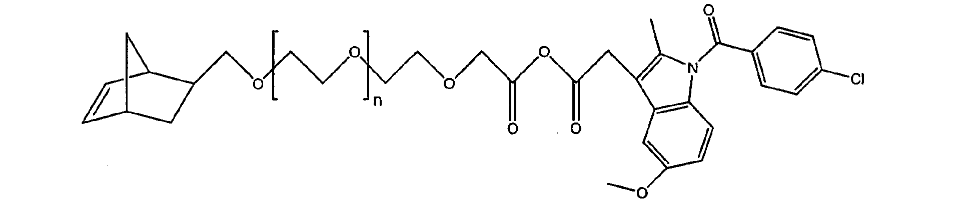

avec n compris entre 70 et 200 en fonction des besoins de l'application envisagée.

Référence : NB-POE-CO(O)-IND.

### Schéma de synthèse :

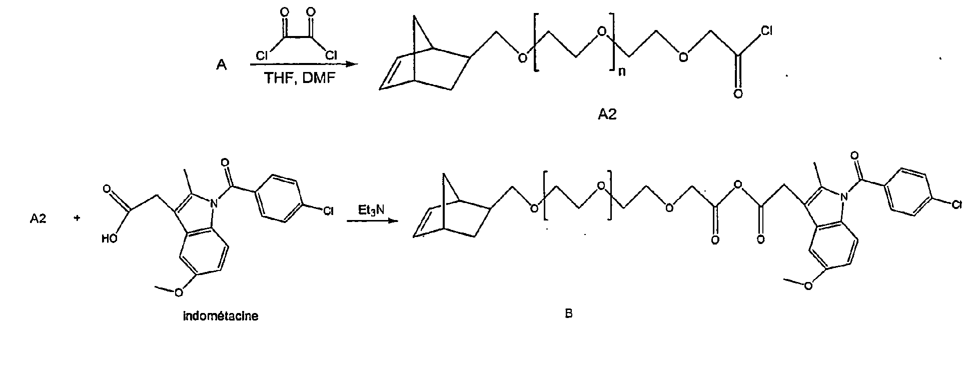

### Procédure de synthèse :

La fonction acide du NB-POE-COOH (A) est transformée en chlorure d'acide (A2) par réaction de A (5,2g) sur le chlorure d'oxalyl (0,08 mL) dans le THF (25mL) en présence d'une quantité catalytique de diméthylformamide pendant 24h. L'indométacine (0,6g) ainsi que la triéthylamine (0,24mL) sont alors additionnés à la solution de A2 et laisser sous agitation pendant 15h. Après précipitation dans l'éther, le macromonomère B est obtenu.

### 3. Dérivé indométacine du norbornène

Le monomère utilisé dans les réactions précédentes est le norbornène (NBH), ou le norbornène fonctionnalisé (NBD) par le principe actif. Celui-ci est alors introduit via un pont hydrolysable de type ester, anhydride, amide..... La synthèse du norbornène fonctionnalisé par l'indométacine est décrite ci-après.

### Formule chimique :

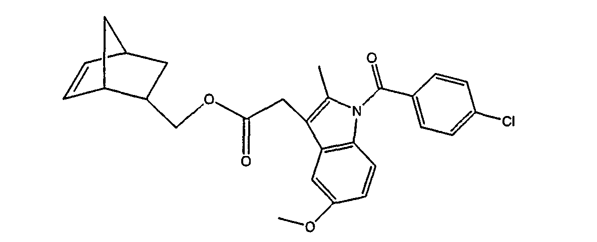

Référence: NBD.

### Schéma de synthèse :

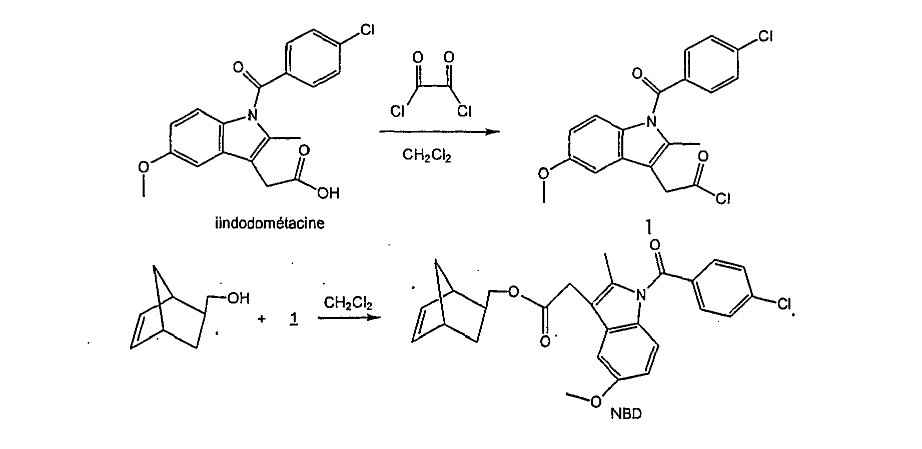

### Synthèse du monomère NBD

Lors d'une réaction type, le chlorure d'oxalyle (0,87mL) est additionné à l'indométacine (1,1g) en solution dans le dichlorométhane (20mL). Après 2 heures de réaction et élimination du chlorure d'oxalyle non réagi, le composé 1 obtenu est ensuite ajouté à une solution de 5-norbomène-2-méthanol (0,36 mL) dans du dichlorométhane (20mL) en présence de triéthylamine (0, 84mL) et laissé sous agitation pendant 15h à 45°C. Après purification par extraction, le monomère NBD est obtenu (p>95%).

### B. Synthèse des particules

Les particules de l'invention sont obtenues par copolymérisation en milieu dispersé (émulsion, mini- et micro-émulsion, dispersion, suspension) de monomères vinyliques (cyclo-oléfines) avec des macromonomères α,ω-fonctionnalisés par une entité polymérisable et une fonction réactive ou un principe actif (médicaments, molécules organiques...). La polymérisation est amorcée par des métaux de transition et peut se faire en milieu aqueux ou organique (dichlorométhane/éthanol). Les macromonomères jouent le rôle de stabilisant et d'agent de fonctionnalisation. En tant que stabilisants ils permettent, lors de la formation du polymère dans le milieu réactionnel, de le disperser sous forme de nanoparticules sphériques. Purement stérique la stabilisation est insensible à toute variation de pH du milieu. Par ailleurs, la fonctionnalisation du latex par l'intermédiaire de macromonomère améliore la disponibilité des fonctions réactives à la surface du latex et préserve leur réactivité.

L'amorceur de la polymérisation est un complexe à base de ruthénium stable en milieu polaire : RuCl₃, RuCl₂(PCy₃)₂CHPh et leurs homologues. Le latex synthétisé dans ces conditions sera constitué de chaînes de polyalcénamère porteuses de greffons poly(oxyde d'éthylène) qui serviront à stabiliser les particules.

Les particules obtenues sont stables en milieu aqueux ou /et organique. Leur taille est comprise entre quelques nanomètres et quelques micromètres en fonction du procédé de polymérisation (dispersion, suspension, mini-émulsion...) utilisé. Les nanoparticules sont sphériques et de très bonne isométrie.

### Schéma de synthèse :

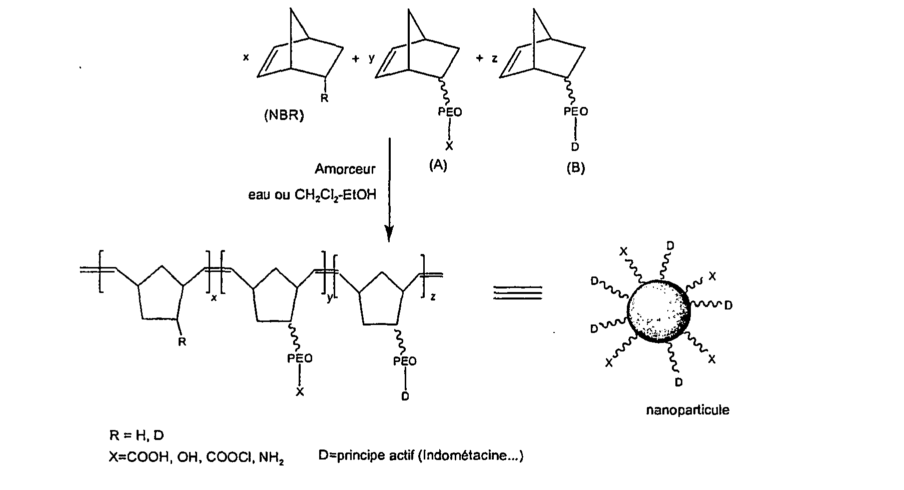

### Procédure de synthèse :

Les macromonomères A et B sont copolymérisés en présence de monomère (NBH et/ou NBD). Dans une réaction type, 0,8g de monomère et 1g de macromonomère (0,2g de A et 0,8g de B) préalablement dissous dans 14ml d'un mélange dichlorométhane/éthanol (35%/65%) sont ajoutés sous azote et sous vive agitation à 10ml de dichlorométhane/éthanol (50%50%) contenant 20mg d'amorceur. La durée de la polymérisation est de une heure. Le milieu totalement homogène au départ devient de plus en plus turbide au fur et à mesure que la polymérisation a lieu. Le suivi des polymérisations, par chromatographie gazeuse, a révélé des conversions des monomères totales en moins de une minute. L'incorporation des macromonomères A et B dans le latex est totale.

### C. VARIANTE POUR LE TRANSPORT DE PRINCIPE ACTIF SENSIBLE.

Le latex est réalisé comme précédemment par copolymérisation entre une cyclo-oléfine (le norbomène) porteuse d'un principe actif (Indométacine) ou pas et le stabilisant polymère (NB-PCL-POE-OMe). Ce dernier fonctionnalisé ou pas par une fonction réactive de type acide, chlorure d'acide, alcool, amine (même fonction que précédemment) possède un pont hydrolysable, notamment des motifs d'ε-caprolactone (PCL) entre la fonction polymérisable et la chaîne de polyoxyde d'éthylène selon le schéma ci-après.

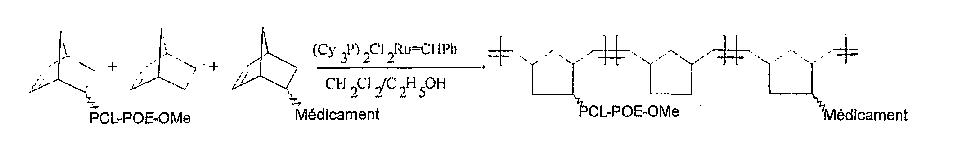

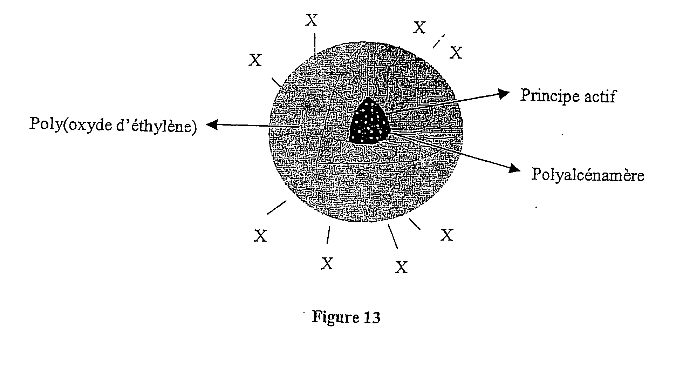

Suivant cette stratégie la libération du principe actif, emprisonné à l'intérieur de la particule et lié de manière covalente à celle-ci (Figure 13), nécessite une première étape de déstabilisation du latex. Celle-ci peut être réalisée via un stimuli extérieur (pH, hyperthermie...) par relargage des chaînes stabilisantes.

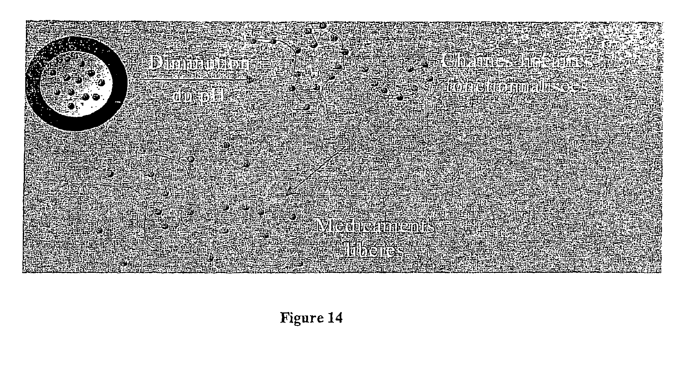

Les chaînes linéaires de polyalcénamères résultantes fonctionnalisées par le principe actif subissent dans un deuxième temps des réactions d'hydrolyses et libèrent le principe actif (Figure 14).

### 1) Procédure pour la synthèse copolymère poly(caprolactone-β-éthylène glycol)-α-norbornène-ω-méthyléther NB-PCL-POE-OMe

### Préparation du poly(caprolactone)α-norbornènyle (NB-Pcapro)

A une solution de 2-hydroxyméthyl-5-norbornène (1,3.10⁻² mole) dans le toluène (100 mL) refroidie à -80°C est ajouté le triéthylaluminium (1,3.10⁻² mole) goutte à goutte. Après un retour progressif à température ambiante, la réaction est poursuivie pendant 2,5 heures. La caprolactone (3,9 mole) est ensuite additionnée au milieu réactionnel sous agitation vigoureuse. Après 18 heures de réaction, 50 mL d'acide chlorhydrique (0,1 N) sont ajoutés. Après un lavage à neutralité, le poly(ε-caprolactone)-α-norbornènyle est précipité à froid dans l'heptane puis filtré sur fritté n°4. Les traces d'heptane seront éliminés par un chauffage (40°C) sous vide pendant 10 heures. Le polymère obtenu est ensuite lyophilisé 3 fois avec du dioxane en tant que solvant.

### Préparation du poly(éthylène glycol)-α-acide carboxylique-ω-méthyléther

Solubiliser 3,89.10⁻³ mole d'anhydride succinique et 4.10⁻³ mole de triéthylamine dans 45 mL d'acétone anhydre. Ajouter sous agitation et goutte à goutte une solution de poly(éthylène glycol) monométhyléther (6.10⁻⁴ mole) dans 15 mL de CH₂Cl₂ anhydre. Après 16 heures de réaction, additionner 1 mL de méthanol. Après concentration à l'évaporateur rotatif, précipiter le polymère dans l'éther éthylique. Recommencer 2 autres fois les étapes de dissolution/précipitation. Placer le polymère sous vide dynamique pendant 10 heures pour éliminer toutes traces de solvant.

### Préparation du copolymère poly(caprolactone-b-éthylène glycol)-α-norbornène-ω-méthyléther (NB-Pcapro-PEG-OMe)

Solubiliser 4.10⁻⁴ mole de poly(éthylène glycol)-α-acide carboxylique-ω-méthyléther dans 40 mL de CH₂Cl₂ anhydre. Ajouter à cette solution refroidie à 5°C le chlorure d'oxalyle (8.10⁻⁴ mole). Après 15 heures de réaction, enlever l'excès de chlorure d'oxalyle non réagi ainsi que le CH₂Cl₂ sous pression réduite. Le résidu jaune obtenu est ensuite redissous dans 40 mL de dichlorométhane. Après avoir additionné la triéthylamine (4,3.10⁻⁴ mole), ajouter goutte à goutte et sous agitation le poly(caprolactone) α-norbornènyle. Après concentration à l'évaporateur rotatif, précipiter le polymère dans l'éther éthylique. Recommencer 2 autres fois les étapes de dissolution/précipitation. Placer le polymère sous pression réduite pendant 10 heures pour éliminer toutes traces de solvant.

La synthèse du poly(ε-caprolactone)α-norbornényle est effectuée selon le schéma suivant :

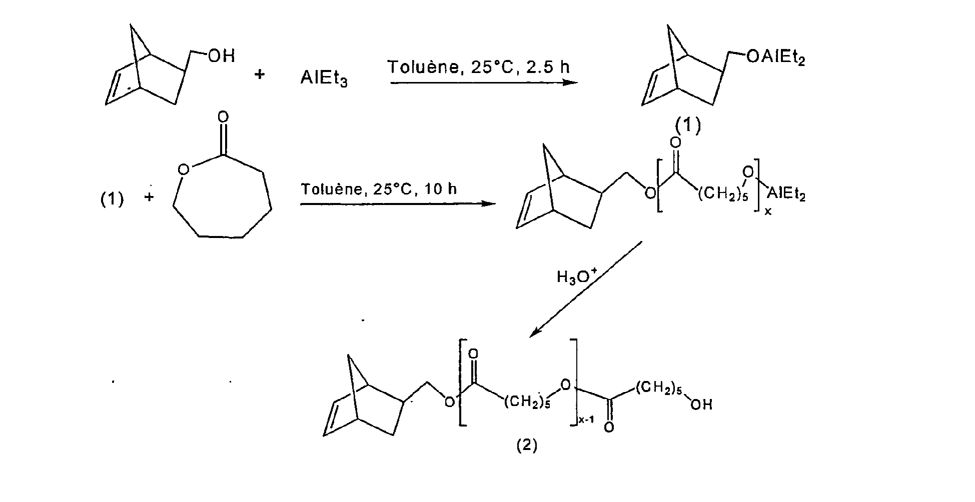

La synthèse du poly(ε-caprolactone-b-éthylène glycol)-α-norbornène-ω-méthyléther est effectuée selon le schéma suivant :

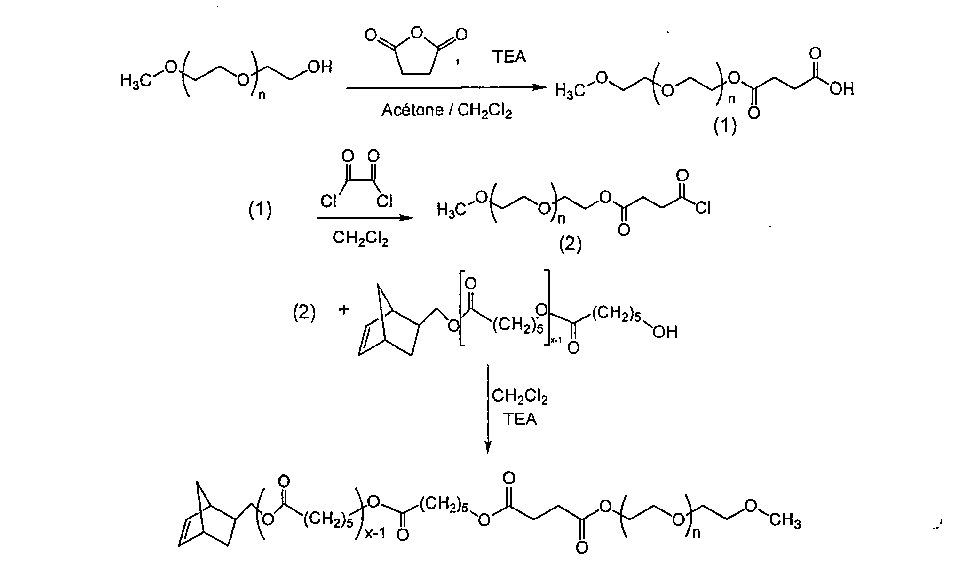

### 2) Libération du principe actif Une fois la particule synthétisée, nous avons vérifié par spectrométrie UV-Visible la possibilité de libérer le médicament par simple abaissement du pH. Les résultats obtenus ont permis de confirmer une libération progressive et contrôlée de l'indométacine. Par ailleurs, l'application d'un pH égal à 3 a révélé que plus de 85% de celui-ci pouvait être relargué en 48h

### Légendes des figures

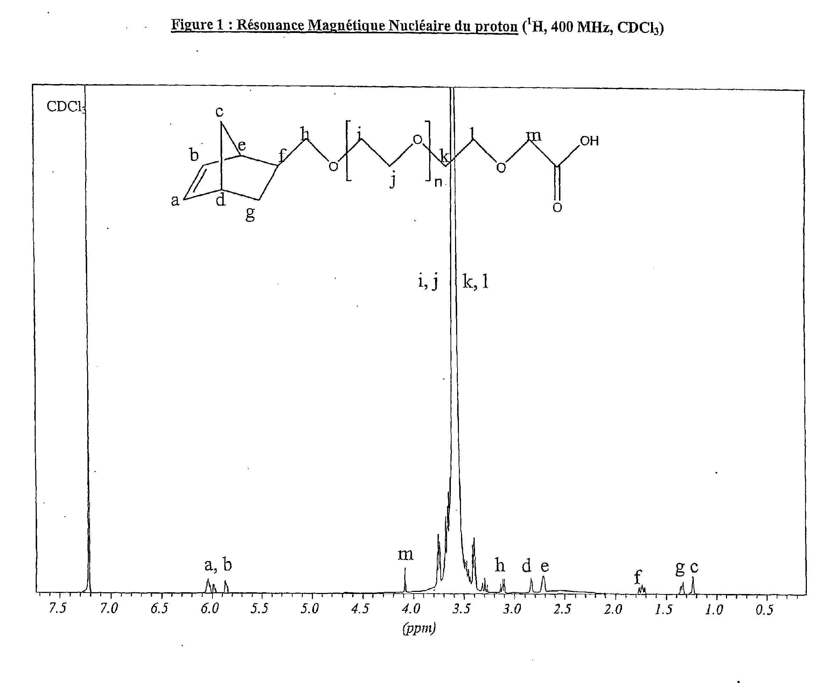

- Figure 1 : Spectre RMN ¹H du macromonomère de formule A.

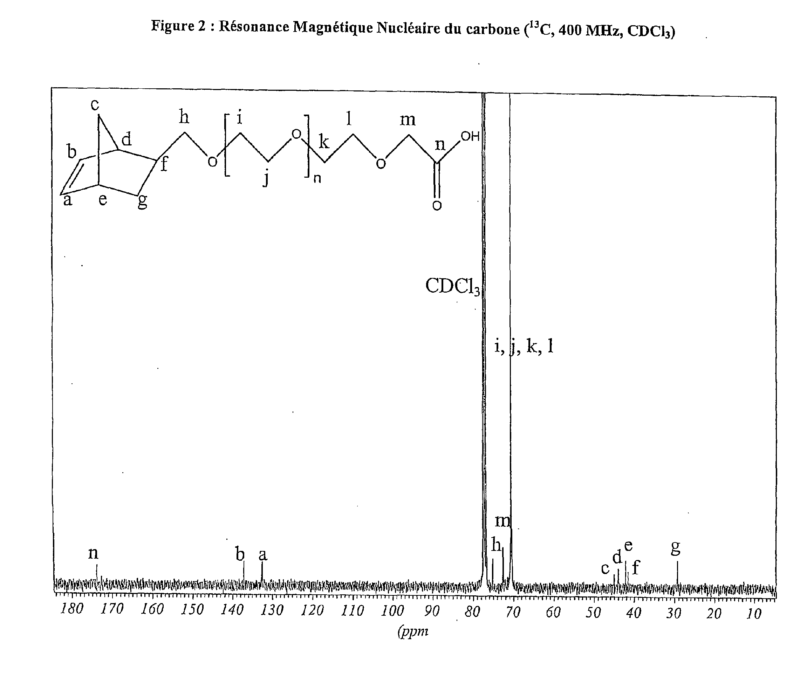

- Figure 2 : Spectre RMN ¹³C du macromonomère de formule A.

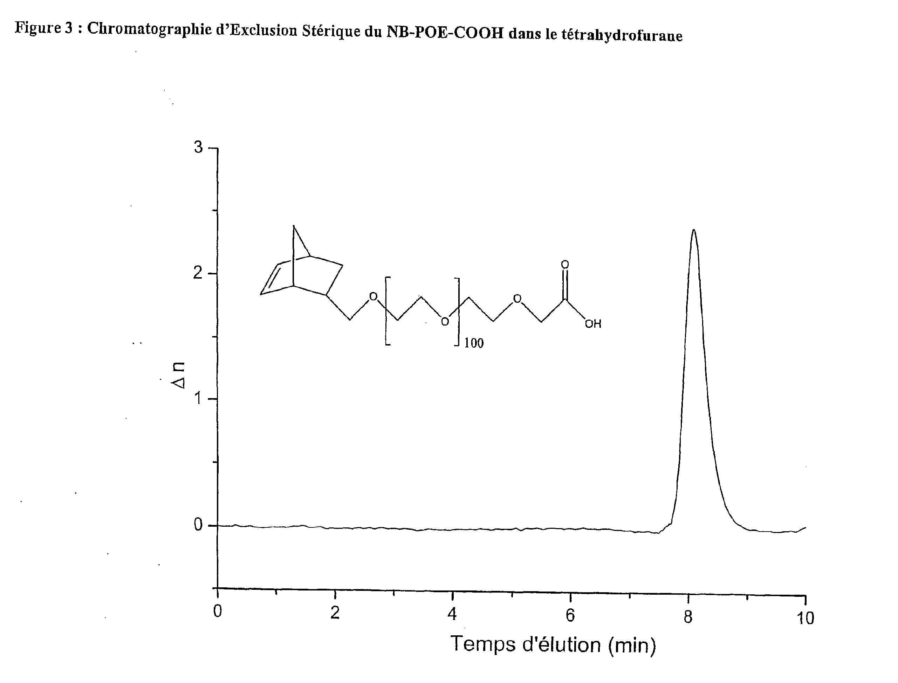

- Figure 3 :Chromatographie d'exclusion stérique du macromonomère de formule A dans le THF.

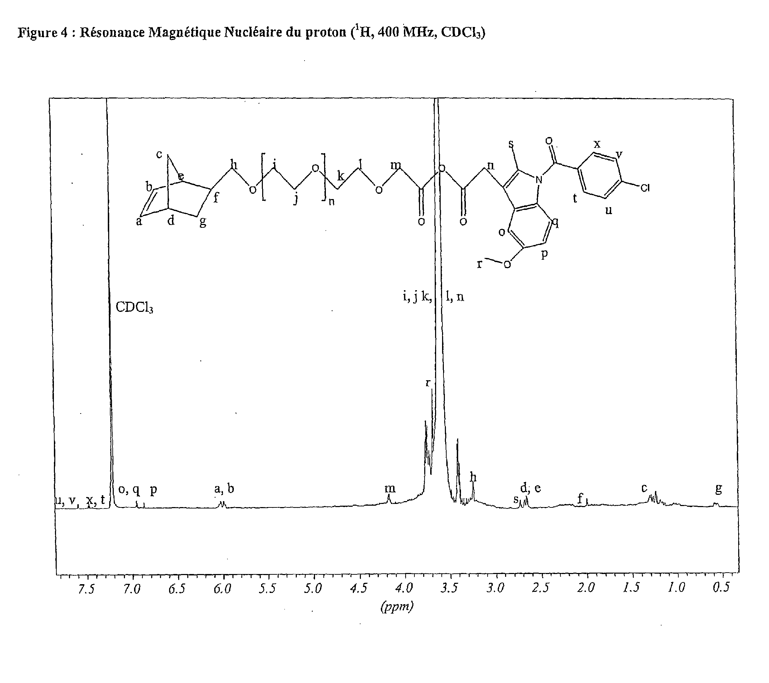

- Figure 4 : Spectre RMN ¹H du macromonomère de formule B.

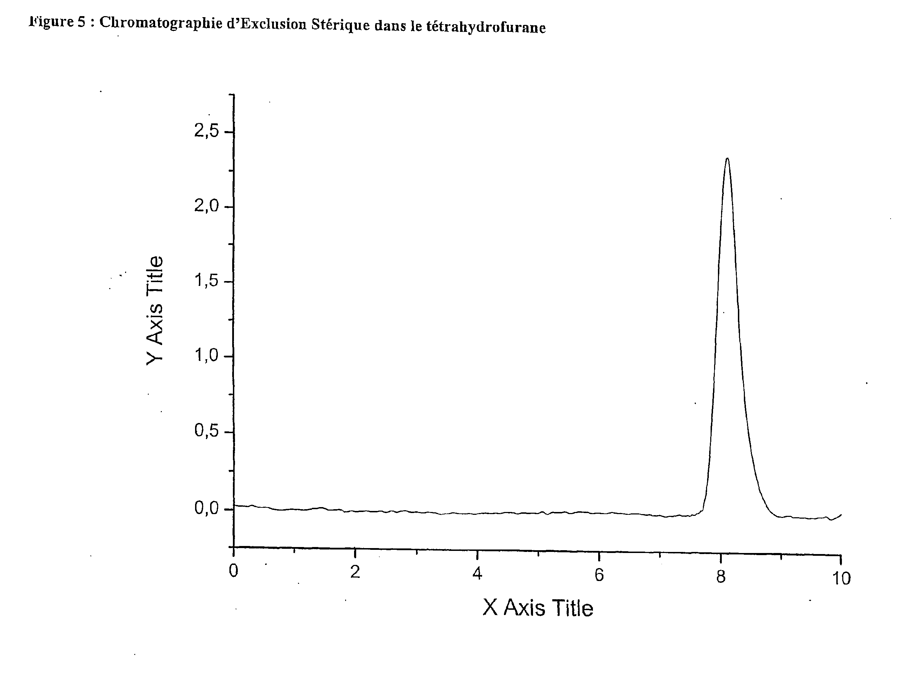

- Figure 5 :Chromatographie d'exclusion stérique du macromonomère de formule B dans le THF.

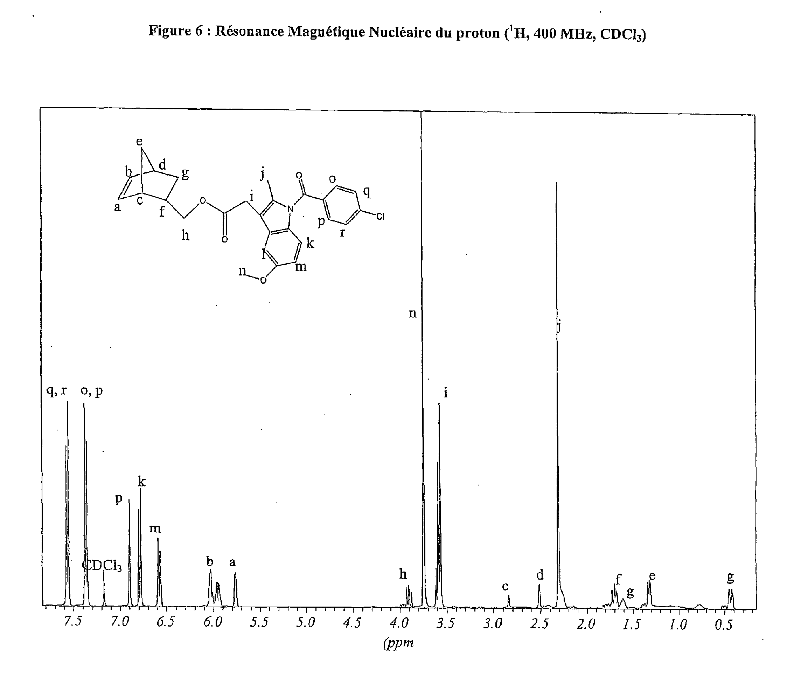

- Figure 6 : Spectre RMN ¹H du composé NBD.

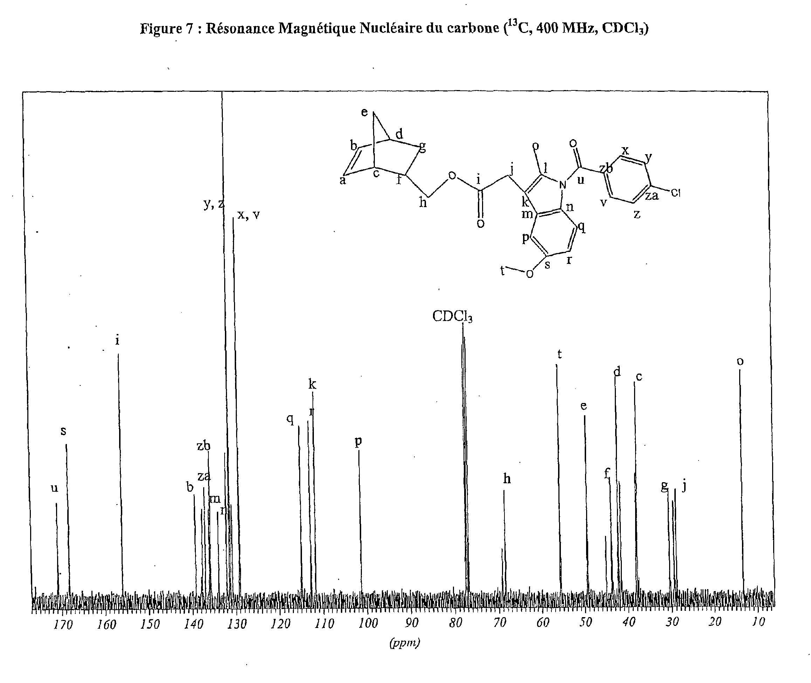

- Figure 7 : Spectre RMN ¹³C du composé NBD.

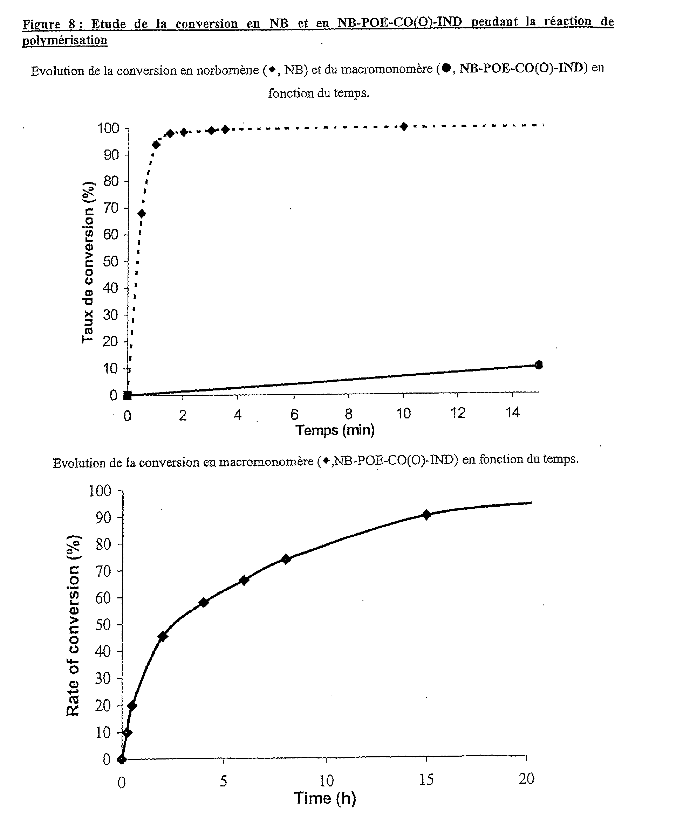

- Figure 8 : Etude de la conversion en NB et en NB-POE-CO(O)-IND pendant la réaction de polymérisation. Evolution de la conversion en norbornène (◆, NB) et du macromonomère (●, NB-POE-CO(O)-IND) en fonction du temps.

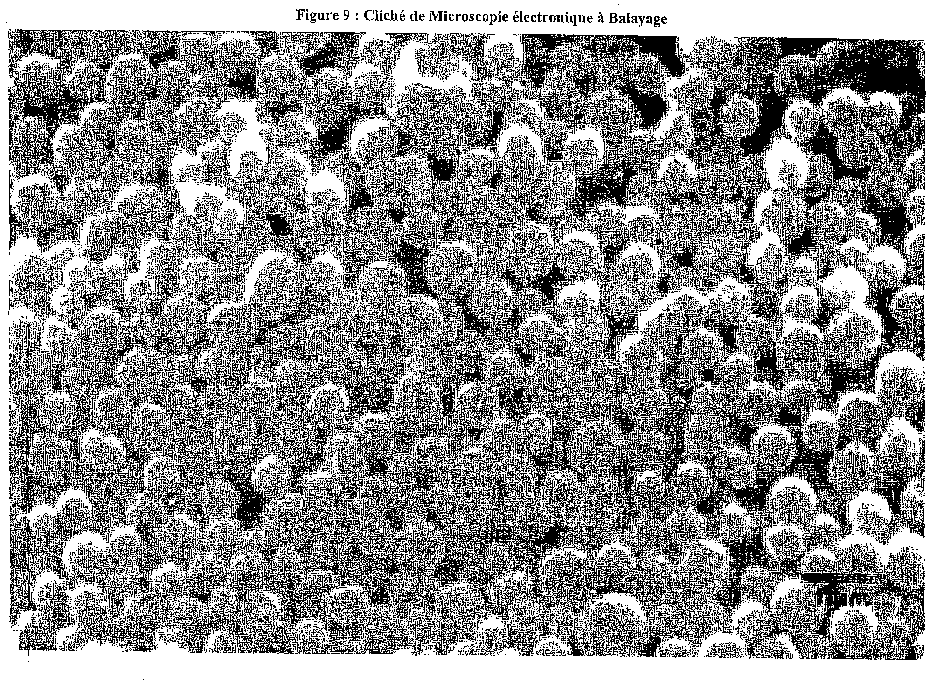

- Figure 9: Cliché de Microscopie électronique à Balayage de particules sphériques obtenues par copolymérisation des macromonomères A et B en présence des monomères NBH et/ou NBD.

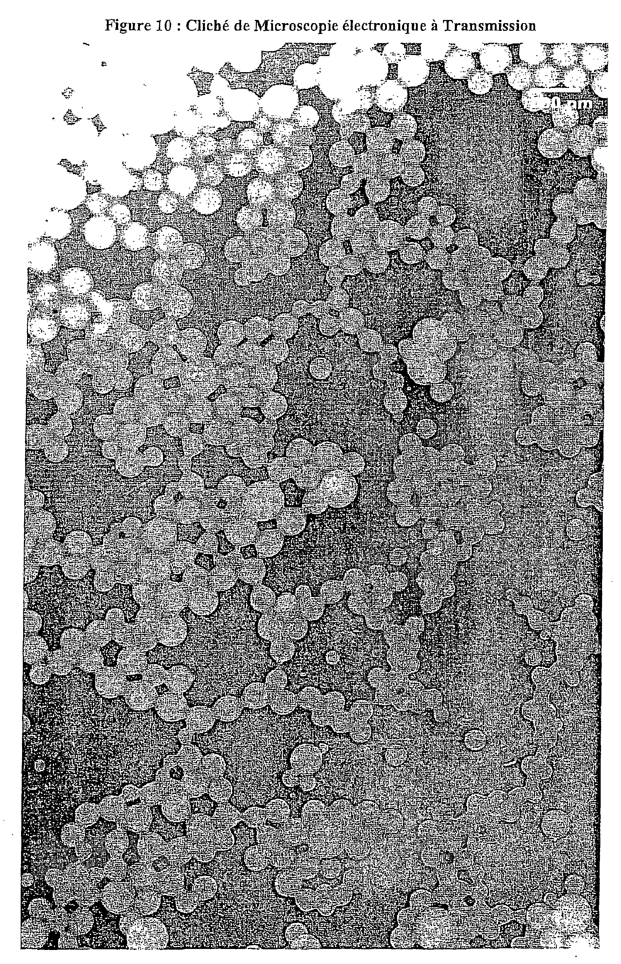

- Figure 10 : Cliché de Microscopie électronique à Transmission de particules sphériques obtenues par copolymérisation des macromonomères A et B en présence des monomères NBH et/ou NBD.

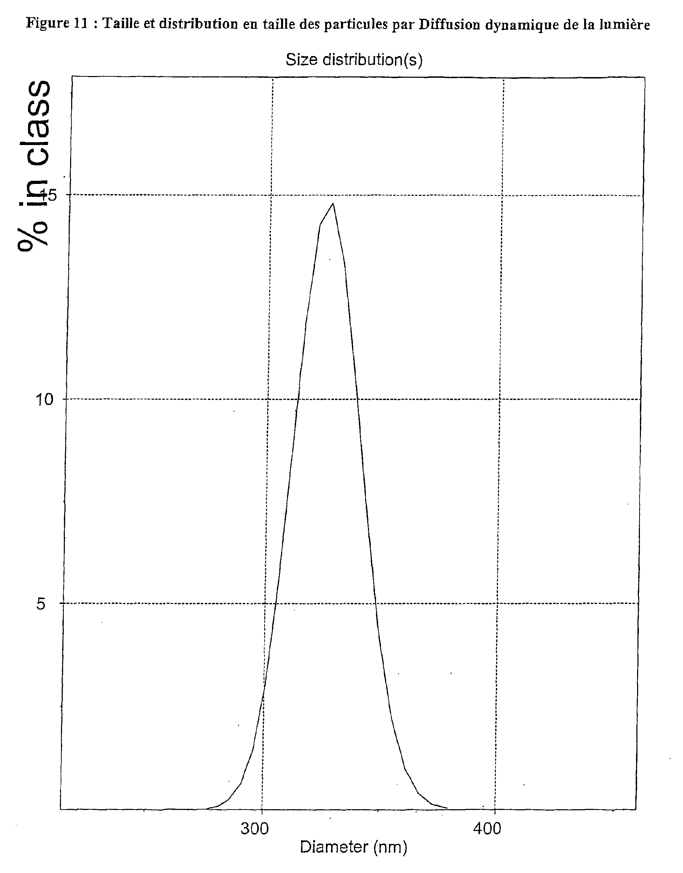

- Figure 11 : Taille et distribution en taille des particules sphériques obtenues par copolymérisation des macromonomères A et B en présence des monomères NBH et/ou NBD, par diffusion dynamique de la lumière.

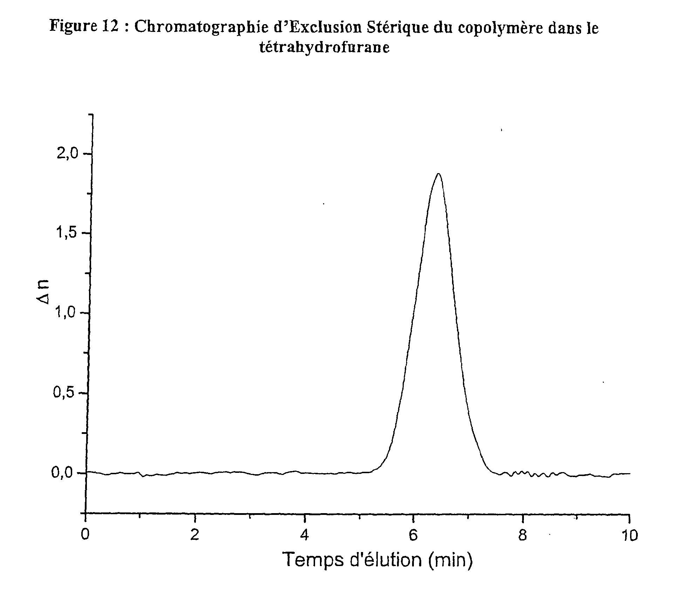

- Figure 12 :Chromatographie d'exclusion stérique des particules sphériques obtenues par copolymérisation des macromonomères A et B en présence des monomères NBH et/ou NBD dans le THF. - Figure 13 : Représentation d'une particule sphérique selon l'invention dans laquelle le principe actif est emprisonné à l'intérieur de la particule et lié de manière covalente à celle-ci.
- Figure 14 : Illustration de la déstabilisation d'une particule sphérique selon l'invention (ou latex) et relargage du médicament.

## Revendications

1. Particules sphériques d'un diamètre compris entre 10 nm et 100 µm, lesdites particules étant constituées par des chaînes polymère contenant environ 30 à 10000 unités monomères, identiques ou différentes, dérivées de la polymérisation d'alcènes monocycliques dont le nombre d'atomes de carbone constitutifs du cycle est d'environ 4 à 12, ou d'alcènes polycycliques dont le nombre total d'atomes de carbone constitutifs des cycles est d'environ 6 à 20, l'une au moins desdites unités monomères étant substituée par une chaîne R comprenant un polyoxyde d'éthylène de formule (A) le cas échéant lié de façon covalente auxdites unités monomères via un pont hydrolysable
-(CH₂-CH₂-O)ₙ-X (A)
dans laquelle n représente un nombre entier d'environ 50 à 340, notamment de 70 à 200, et X représente une chaîne alkyle ou alkyloxy d'environ 1 à 10 atomes de carbone, comprenant une fonction réactive du type OH, halogène, NH₂, C(O)X₁ dans laquelle X₁ représente un atome d'hydrogène, d'halogène, un groupe OR' ou NHR' dans lequel R' représente un atome d'hydrogène ou une chaîne hydrocarbonée d'environ 1 à 10 atomes de carbone, substituée ou non, ou X représente un groupe comprenant une fonction photosensible telle que les polyènes, ladite fonction réactive étant le cas échéant engagée dans une liaison avec un principe actif, ou une molécule biologique telle qu'une protéine, ladite chaîne R étant liée de façon covalente auxdites unités monomères.

2. Particules sphériques selon la revendication 1, **caractérisées en ce que** les unités monomères sont dérivées de la polymérisation d'alcènes monocycliques, et sont de formule (Z1) suivante
=[CH-R₁-CH]= (Z1)
dans laquelle R₁ représente une chaîne hydrocarbonée de 2 à 10 atomes de carbone, saturée ou non, lesdites unités monomères étant le cas échéant substituées par une chaîne R, ou directement par un groupe X, tels que définis dans la revendication 1.

3. Particules sphériques selon la revendication 1 ou 2, **caractérisées en ce que** les alcènes monocycliques dont sont issues les unités monomères sont :
- le cyclobutène conduisant à un polymère comprenant des unités monomères de formule (Z1a) suivante : 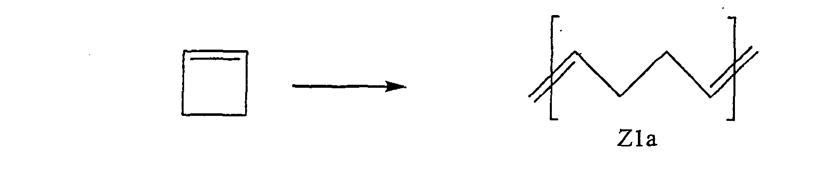
- le cyclopentène conduisant à un polymère comprenant des unités monomères de formule (Z1b) suivante : 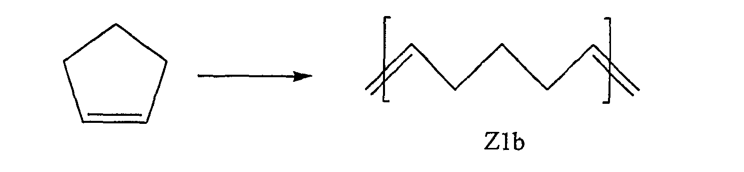
- le cyclopentadiène conduisant à un polymère comprenant des unités monomères de formule (Z1c) suivante 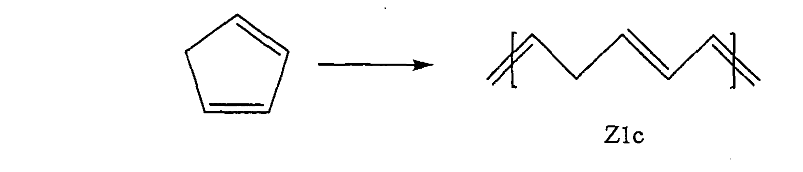
- le cyclohexène conduisant à un polymère comprenant des unités monomères de formule (Z1d) suivante 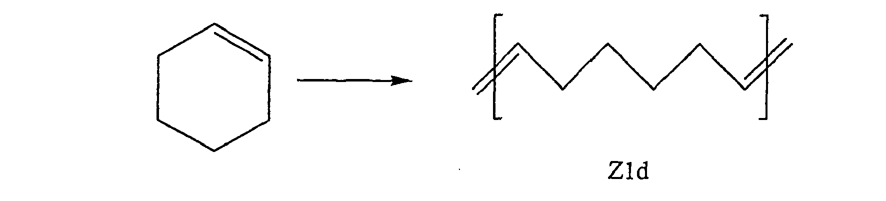
- le cyclohexadiène conduisant à un polymère comprenant des unités monomères de formule (Z1e) suivante 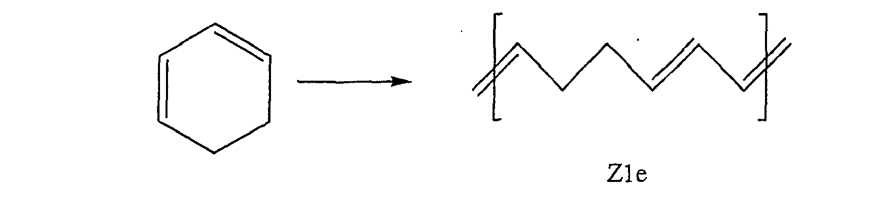
- le cycloheptène conduisant à un polymère comprenant des unités monomères de formule (Z1f) suivante 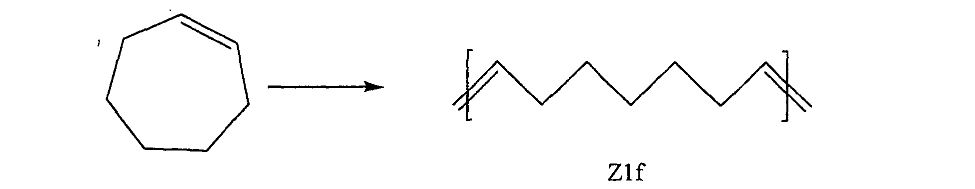
- le cyclooctène conduisant à un polymère comprenant des unités monomères de formule (Z1h) suivante 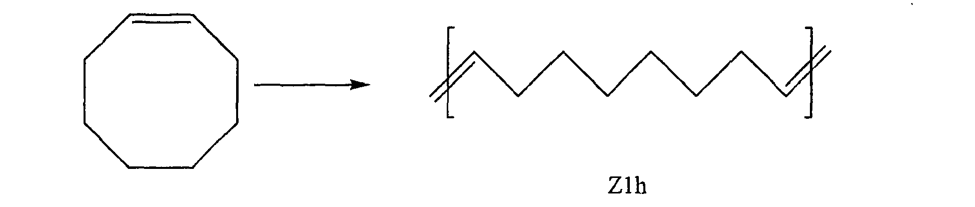
- le cyclooctapolyène, notamment le cycloocta-1,5-diene conduisant à un polymère comprenant des unités monomères de formule (Zli) suivante 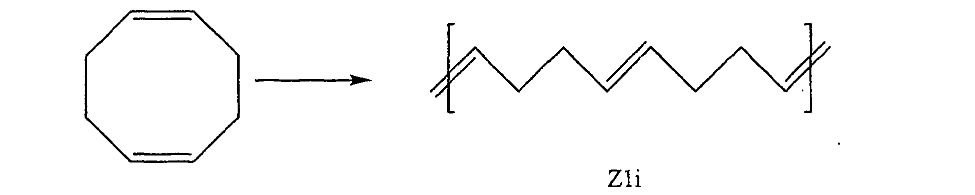
- le cyclononène conduisant à un polymère comprenant des unités monomères de formule (Z1j) suivante 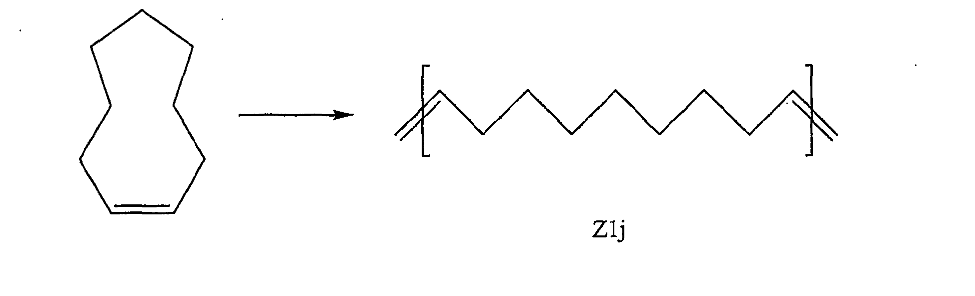
- le cyclononadiène conduisant à un polymère comprenant des unités monomères de formule (Z1k) suivante 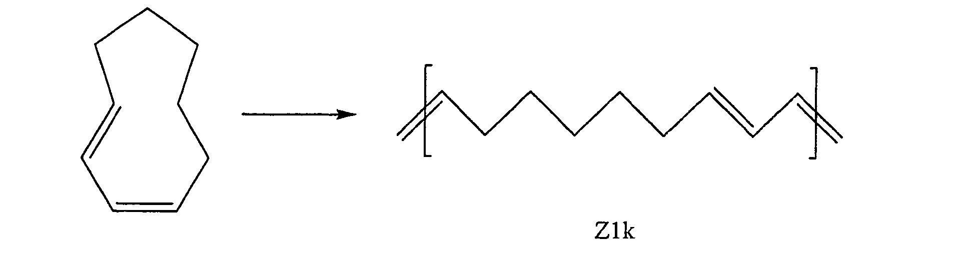
- le cyclodécène conduisant à un polymère comprenant des unités monomères de formule (Z1l) suivante 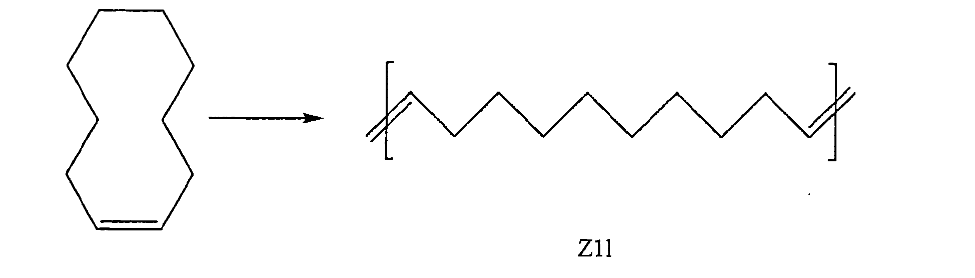
- le cyclodéca-1,5-diène conduisant à un polymère comprenant des unités monomères de formule (Z1m) suivante 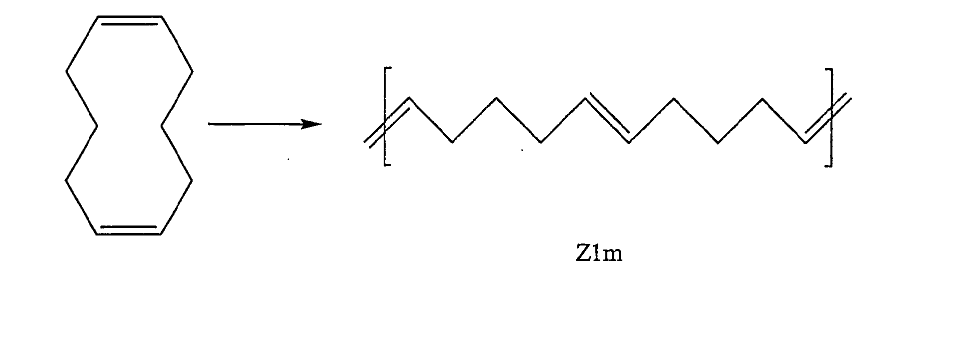
- le cyclododécène conduisant à un polymère comprenant des unités monomères de formule (Z1n) suivante 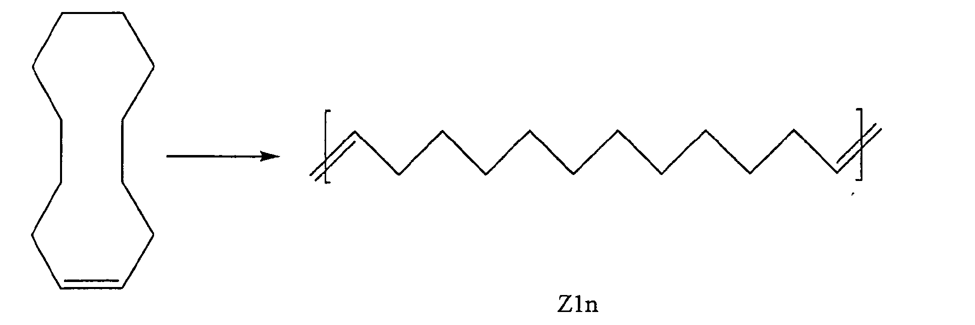
- ou encore l'acétate du 2,3,4,5-tétrahydrooxepin-2-yl, le cyclopentadécène, le paracyclophane, le ferrocenophane.

4. Particules sphériques selon la revendication 1, **caractérisées en ce que** les unités monomères sont dérivées de la polymérisation d'alcènes polycycliques, et sont :
- de formule (Z2) suivante
=[CH-R₂-CH]= (Z2)
dans laquelle R₂ représente :
* un cycle de formule 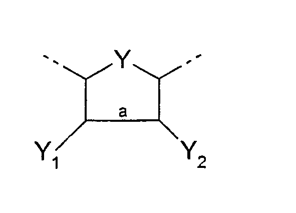 dans laquelle :
. Y représente -CH₂-, ou un hétéroatome, ou un groupe -CHR-, ou un groupe -CHX-, R et X étant tels que définis dans la revendication 1,
. Y₁ et Y₂, indépendamment l'un de l'autre, représentent H, ou une chaîne R, ou un groupe X susmentionnés, ou forment en association avec les atomes de carbone qui les portent un cycle de 4 à 8 atomes de carbone, ce cycle étant le cas échéant substitué par une chaîne R, ou un groupe X susmentionnés,
. a représente une simple ou double liaison,
* ou un cycle de formule
dans laquelle : 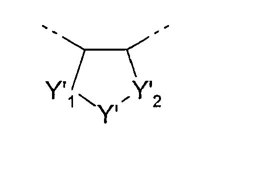
. Y' représente -CH₂-, ou un hétéroatome, ou un groupe -CHR-, ou un groupe -CHX-, R et X étant tels que définis ci-dessus,
. Y'₁ et Y'₂, indépendamment l'un de l'autre, représentent -CH₂-, ou un groupe -C(O), ou un groupe -COR, ou un groupe -C-OX, R et X étant tels que définis ci-dessus,
- de formule (Z3) suivante 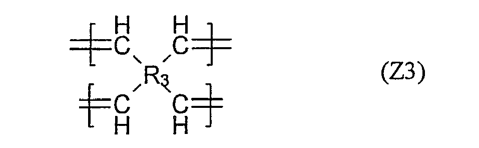 dans laquelle R₃ représente :
* un cycle de formule 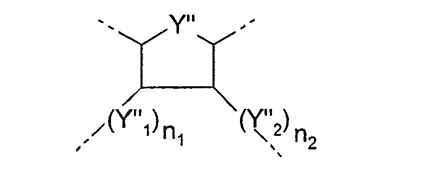 dans laquelle :
. n₁ et n₂, indépendamment l'un de l'autre, représentent 0 ou 1,
. Y" représente -CH₂-, ou un groupe -CHR-, ou un groupe -CHX-, R et X étant tels que définis ci-dessus,
. Y"₁ et Y"₂, indépendamment l'un de l'autre, représentent une chaîne hydrocarbonée de 0 à 10 atomes de carbone,
* ou un cycle de formule 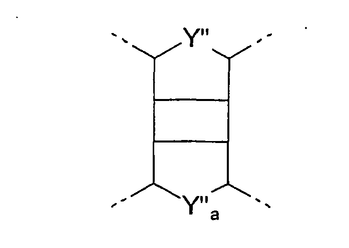 dans laquelle Y" et Y"ₐ, indépendamment l'un de l'autre, représentent -CH₂-, ou un groupe -CHR-, ou un groupe -CHX-, R et X étant tels que définis ci-dessus,
* ou un cycle de formule 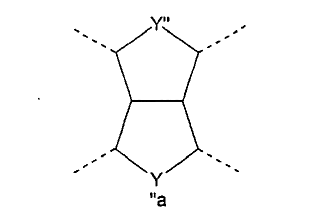 dans laquelle Y" et Y"ₐ, indépendamment l'un de l'autre, représentent -CH₂-, ou un groupe -CHR-, ou un groupe -CHX-, R et X étant tels que définis ci-dessus.

5. Particules sphériques selon la revendication 1 ou 4, **caractérisées en ce que** les alcènes polycycliques dont sont issues les unités monomères sont :
- les monomères contenant un cycle cyclobutène conduisant à un polymère comprenant des unités monomères de formule (Z2a) suivante : 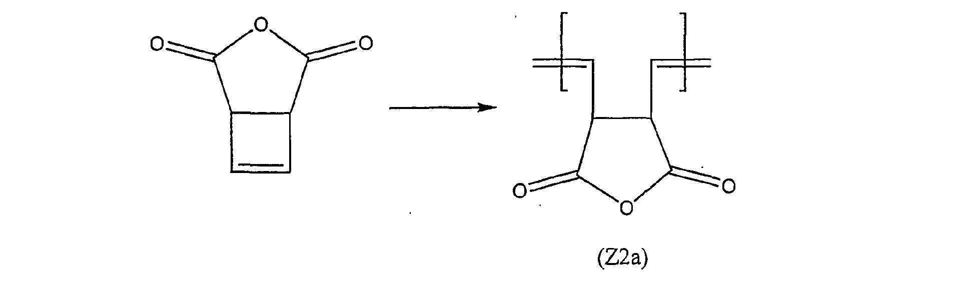
- les monomères contenant un cycle cyclopentène conduisant à un polymère comprenant des unités monomères de formule (Z2b) suivante : 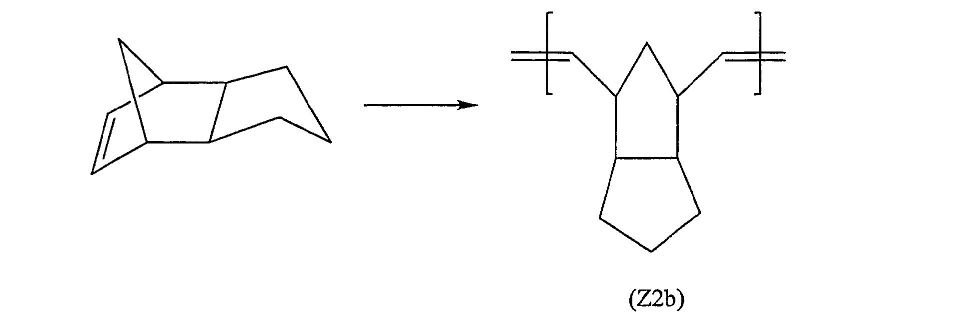
- le norbornène (bicyclo[2.2.1]hept-2-ène) conduisant à un polymère comprenant des unités monomères de formule (Z2c) suivante : 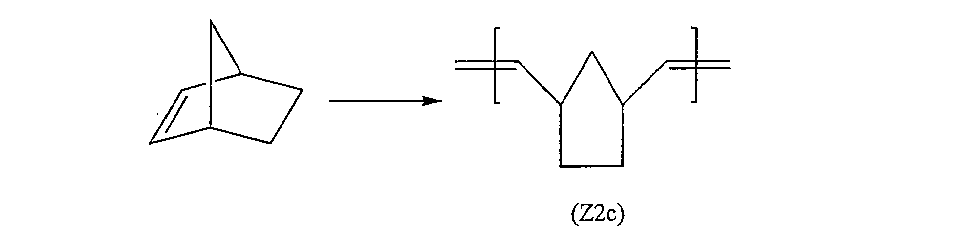
- le norbornadiène conduisant à un polymère comprenant des unités monomères de formule (Z2d) suivante : 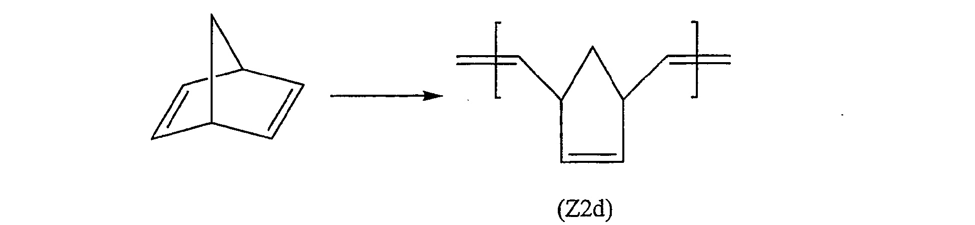
- le 7-oxanorbornène conduisant à un polymère comprenant des unités monomères de formule (Z2e) suivante : 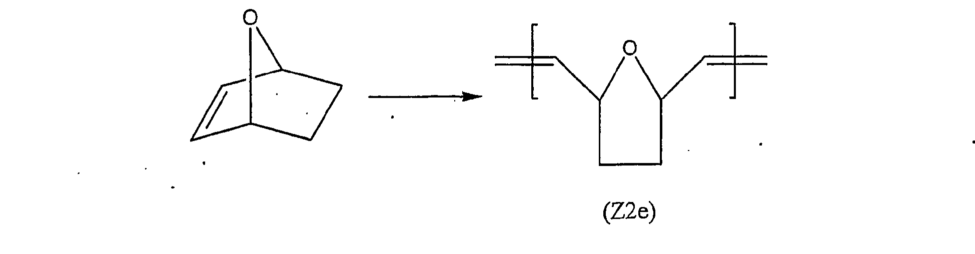
- le 7-oxanorbornadiène conduisant à un polymère comprenant des unités monomères de formule (Z2f) suivante : 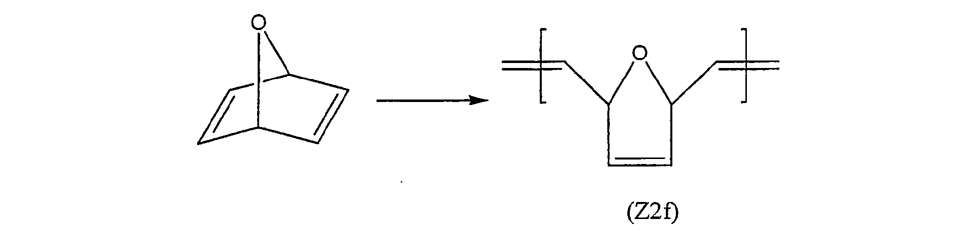
- le dimère du norbornadiène conduisant à un polymère comprenant des unités monomères de formule (Z3a) suivante : 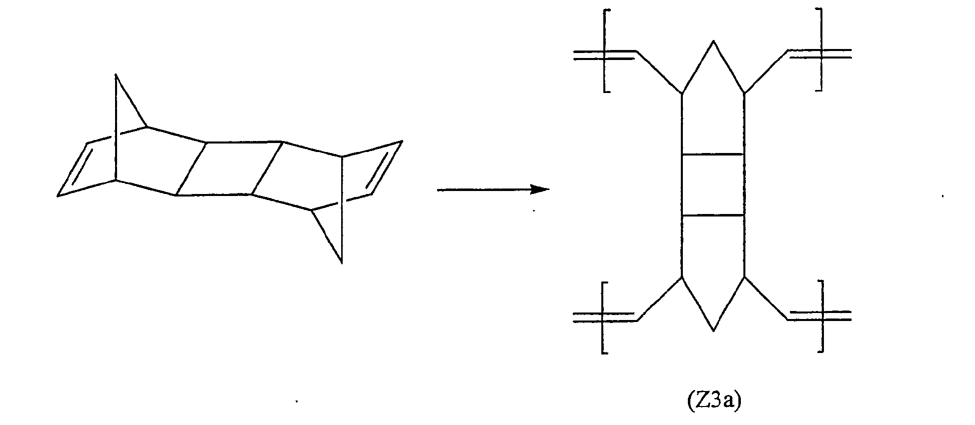
- le dicyclopentadiène conduisant à un polymère comprenant des unités monomères de formule (Z3b) suivante : 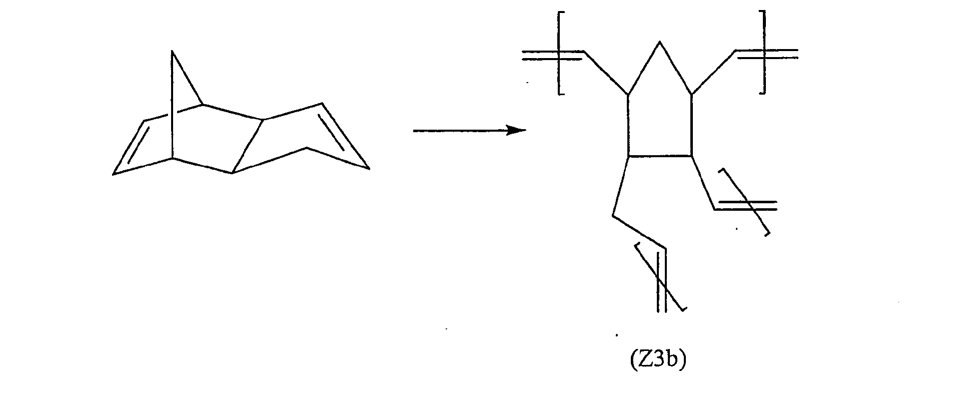
- le tetracyclododécadiene conduisant à un polymère comprenant des unités monomères de formule (Z3c) suivante : 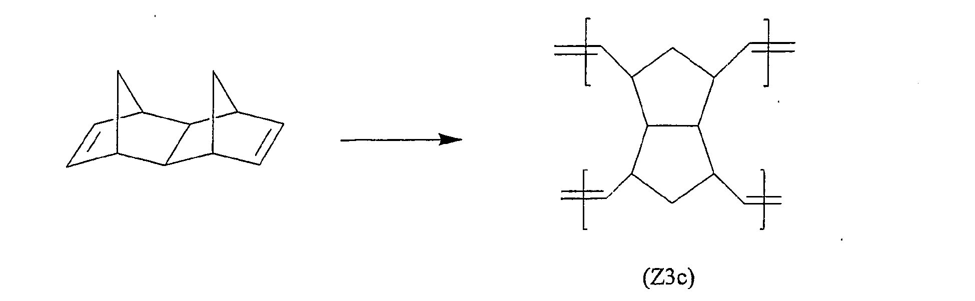
- ou le bicyclo[5.1.0]oct-2-ène, le bicyclo[6.1.0]non-4-ène.

6. Particules sphériques selon l'une des revendications 1 à 5, **caractérisées en ce que** les alcènes mono ou polycycliques dont sont issues les unités monomères sont :
- le norbornène (bicyclo[2.2.1]hept-2-ène) conduisant à un polymère comprenant des unités monomères de formule (Z2c),
- le tetracyclododécadiene conduisant à un polymère comprenant des unités monomères de formule (Z3c),
- le dicyclopentadiène conduisant à un polymère comprenant des unités monomères de formule (Z3b),
- le dimère du norbornadiène conduisant à un polymère comprenant des unités monomères de formule (Z3a),
- le cycloocta-1,5-diene conduisant à un polymère comprenant des unités monomères de formule (Zli).

7. Particules sphériques selon l'une des revendications 1 à 6, **caractérisées en ce que** au moins 0,5 % jusqu'à 100% des unités monomères sont substituées par une chaîne R telle que définie dans la revendication 1.

8. Particules sphériques selon l'une des revendications 1 à 7, **caractérisées en ce qu'**elles comprennent :
- entre environ 0,5% jusqu'à 100% d'unités monomères substituées par une chaîne R telle que définie dans la revendication 1, ladite chaîne R étant identique pour ces monomères,
- et entre environ 0,5% et 99,5% d'unités monomères substituées par une chaîne R telle que définie dans la revendication 1, ladite chaîne R de ces monomères étant différente de la chaîne R des monomères précédents,
- et/ou entre environ 0,5% et 99,5% d'unités monomères substituées directement par un groupe X tel que défini dans la revendication 1, ce groupe X de ces monomères étant identique ou différent du groupe X de la chaîne R des monomères précédents,
- et/ou entre environ 1% et 99,5% d'unités monomères non substituées, le total des pourcentages des différents monomères susmentionnés faisant 100%.

9. Particules sphériques selon l'une des revendications 1 à 8, **caractérisées en ce que** la ou les chaînes R substituant les unités monomères sont représentées par la formule
-CH₂-O-(CH₂-CH₂-O)ₙ- CH₂-CH₂-O-X
dans laquelle n est tel que défini dans la revendication 1, et X représente H, -CH₂-COOH, -CH₂-COCl, ou -CH₂-COY, Y représentant un principe actif, ou une molécule biologique telle qu'une protéine.

10. Particules sphériques selon l'une des revendications 1 à 9, **caractérisées en ce que** la ou les chaînes R comprennent un polyoxyde d'éthylène de formule (A) lié de façon covalente à une particule via un pont hydrolysable choisi parmi les enchaînements d'environ 1 à 10 motifs d'ε-caprolactone, ou de fonctions -OC(O)-, -C(O)OC(O)-, -C(O)-NH-.

11. Particules sphériques selon l'une des revendications 1 à 10, **caractérisées en ce que** la ou les chaînes R comprenant un polyoxyde d'éthylène de formule (A) lié de façon covalente à un pont hydrolysable choisi parmi les enchaînements d'environ 1 à 10 motifs d'ε-caprolactone, sont représentées par la formule
-CH₂-(O-CO-(CH₂)₅)ₜ-O-CO-(CH₂)₅-O-CO-(CH₂)₂-CO-O-(CH₂-CH₂-O)ₙ-(CH₂)₂-O-X
dans laquelle t représente un nombre entier compris entre 1 et 10, et X représente H, -CH₂-COOH, -CH₂-COCl, ou -CH₂-COY, Y représentant un principe actif, ou une molécule biologique telle qu'une protéine.

12. Particules sphériques selon l'une des revendications 1 à 11, **caractérisées en ce que** le principe actif est choisi parmi les molécules utilisées en thérapeutique, cosmétique, parfumerie, ou pour les revêtements de surface, tels que les peintures et les antifoulings.

13. Particules sphériques selon l'une des revendications 1 à 12, **caractérisées en ce que** le principe actif est un médicament utilisé en thérapeutique choisi notamment parmi ceux des classes thérapeutiques suivantes : les anti-inflammatoires, notamment l'indométacine, les anti-cancéreux, les antibiotiques, les anti-coagulants, ou les antimitotiques.

14. Particules sphériques selon l'une des revendications 1 à 12, **caractérisées en ce que** la molécule biologique est choisie parmi les protéines susceptibles de se lier à une cible biologique intra ou extracellulaire, ou à des anticorps, ou à tout autre ligand spécifique.

15. Particules sphériques selon la revendication 14, **caractérisées en ce que** la molécule biologique est choisie parmi les protéines suivantes : l'avidine, l'albumine, les facteurs de croissance tel que le VEGF.

16. Compositions pharmaceutiques comprenant des particules sphériques selon l'une des revendications 1 à 15, lorsque le ou les différents groupes X contiennent un principe actif médicamenteux, le cas échéant en association avec un véhicule pharmaceutiquement acceptable, notamment pour une utilisation sous forme parentérale.

17. Compositions cosmétiques comprenant des particules sphériques selon l'une des revendications 1 à 15, lorsque le ou les différents groupes X contiennent un principe actif utilisé en cosmétique, le cas échéant en association avec un véhicule approprié, notamment pour une application sous forme d'émulsions, crèmes.

18. Compositions de revêtement de surfaces comprenant des particules sphériques selon l'une des revendications 1 à 15, lorsque le ou les différents groupes X contiennent un principe actif utilisé pour les revêtements de surfaces, le cas échéant en association avec un véhicule approprié.

19. Procédé de préparation de particules sphériques définies dans l'une des revendications 1 à 15, **caractérisé en ce qu'**il comprend une étape de polymérisation d'un alcène mono ou polycyclique tel que défini dans l'une des revendications 1 à 5 substitué par une chaîne R telle que définie dans les revendications 1 et 9 à 11, cette étape de polymérisation étant éventuellement effectuée en présence :
- d'un ou plusieurs alcènes mono ou polycycliques tels que définis dans l'une des revendications 1 à 5, identiques ou différents du précédent, et substitués par une chaîne R telle que définie dans la revendication 1 et 9 à 11, ladite chaîne R étant différente de celle substituant l'alcène mono ou polycyclique susmentionné,
- et/ou d'un ou plusieurs alcènes mono ou polycycliques tels que définis dans l'une des revendications 1 à 5, identiques ou différents du précédent, et substitués par un groupe X tel que défini dans la revendication 1, ce groupe X étant identique ou différent du groupe X de la chaîne R des alcènes précédents,
- et/ou d'un ou plusieurs alcènes mono ou polycycliques tels que définis dans l'une des revendications 1 à 5, identiques ou différents des précédents, lesdits alcènes n'étant pas substitués,
ladite polymérisation étant effectuée sous agitation en présence d'un complexe de métal de transition en tant qu'amorceur de la réaction choisi notamment parmi ceux des groupes IV ou VI ou VIII, tel que le ruthénium, l'osmium, le molybdène, le tungstène, l'iridium, le titane, en milieu polaire ou apolaire, notamment à l'aide des complexes à base de ruthénium suivants : RuCl₃, RuCl₂(PCy₃)₂CHPh.

20. Alcènes mono ou polycycliques, **caractérisés en ce qu'**ils sont substitués par une chaîne R, ou un groupe X, tels que définis dans la revendication 1, sous réserve que l'alcène de formule suivante soit exclu : 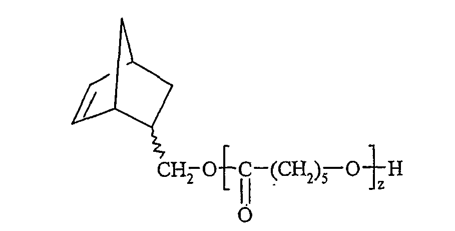 dans laquelle z représente un nombre entier compris entre 1 et 340.

21. Alcènes mono ou polycycliques selon la revendication 20, **caractérisés en ce qu'**ils sont choisis parmi ceux mentionnés dans la revendication 3 ou 5.

22. Alcènes mono ou polycycliques selon la revendication 20 ou 21, **caractérisés par** les formules suivantes : 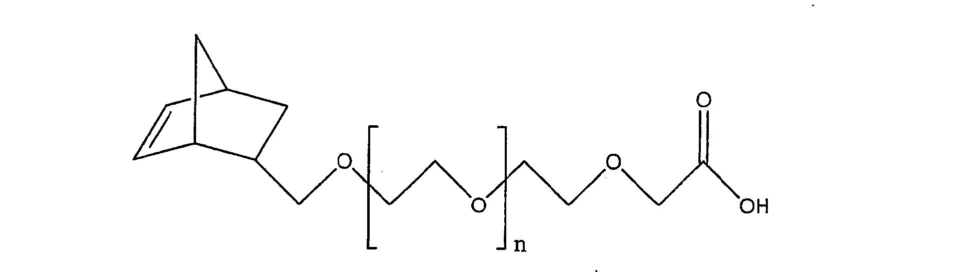 dans laquelle n représente un nombre entier compris entre environ 50 et 340, 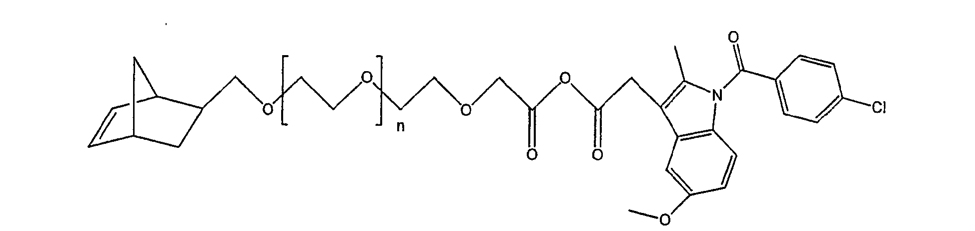 dans laquelle n représente un nombre entier compris entre environ 50 et 340, 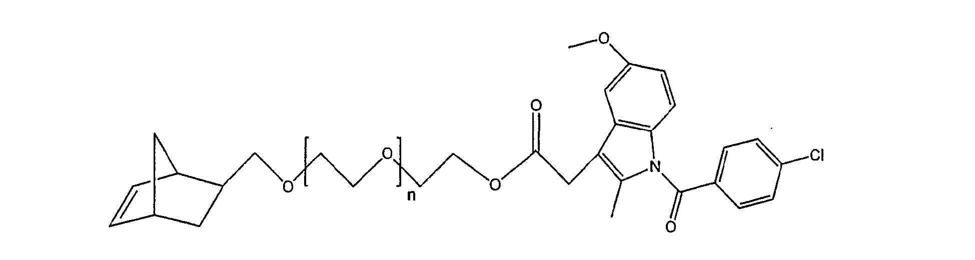 dans laquelle n représente un nombre entier compris entre environ 50 et 340, 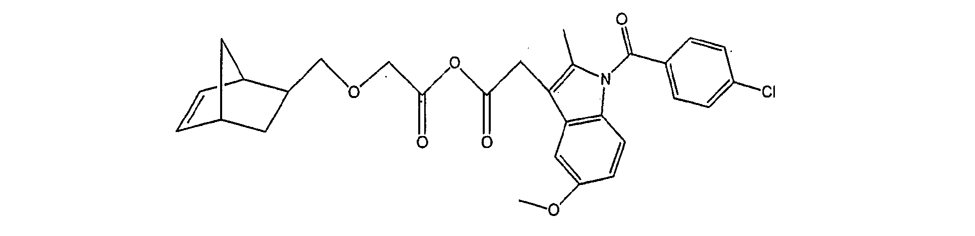 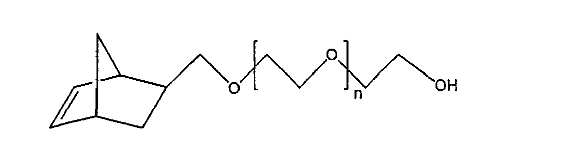 dans laquelle n représente un nombre entier compris entre environ 50 et 340, 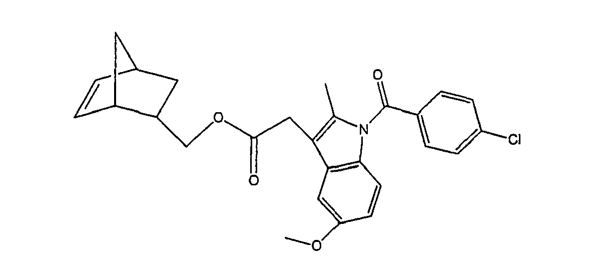 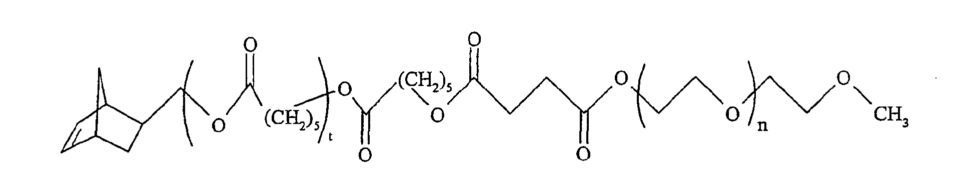 dans laquelle t représente un nombre entier compris entre 1 et 10, et n représente un nombre entier compris entre environ 50 et 340.

23. Utilisation d'alcènes mono ou polycycliques selon l'une des revendications 20 à 22, pour la mise en oeuvre d'un procédé de préparation de particules sphériques définies dans l'une des revendications 1 à 15, notamment selon le procédé de la revendication 19.

## Claims

1. Spherical particles with a diameter ranging between 10 nm and 100 µm, said particles being made up of polymer chains containing approximately 30 to 10000 identical or different monomer units, obtained by polymerising monocyclic alkenes, of which the number of carbon atoms constituting the cycle is approximately 4 to 12, or polycyclic alkenes, the total number of carbon atoms of which constituting the cycle is approximately 6 to 20, at least one of said monomer units being substituted by a chain R containing an ethylene polyoxide based on formula (A) covalently bonded, depending on the case, to said monomer units via a hydrolysable bridge,
-(CH₂-CH₂-O)ₙ-X (A)
In which n stands for a whole number of approximately 50 to 340, in particular 70 to 200, and X stands for an alkyl or alkyloxy chain with approximately 1 to 10 carbon atoms, containing a reactive function of the OH, halogen, NH₂, C(O)X₁ type in which X₁ stands for a hydrogen atom, a halogen atom, an OR' or NHR' group in which R' stands for a hydrogen atom or a hydrocarbonated chain of approximately 1 to 10 carbon atoms, which may or may not be substituted, where X stands for a group containing a photo-sensitive function such as polyenes, said reactive function being involved in a bond with an active ingredient or a biological molecule such as a protein, depending on the case, said chain R being covalently bonded to said monomer units.

2. Spherical particles as claimed in claim 1, **characterised in that** the monomer units are obtained by polymerising monocyclic alkenes and are based on formula (Z1) below
=[CH-R₁-CH]= (Z1)
in which R₁ stands for a hydrocarbonated chain with 2 to 10 carbon atoms, which may or may not be saturated, said monomer units being substituted, depending on the case, by a chain R or directly by a group X as defined in claim 1.

3. Spherical particles as claimed in claim 1 or 2, **characterised in that** the monocyclic alkenes from which the monomer units are obtained are:
- cyclobutene leading to a polymer containing monomer units based on formula (Z1a) below: 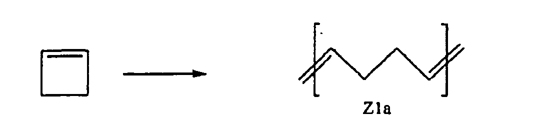
- cyclopentene leading to a polymer containing monomer units based on formula (Z1b) below: 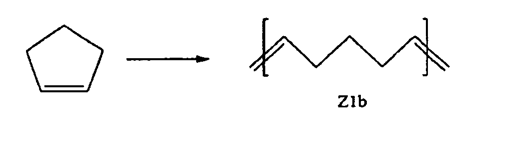
- cyclopentadiene leading to a polymer containing monomer units based on formula (Z1c) below: 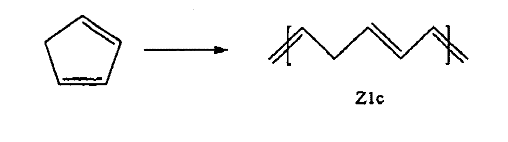
- cyclohexene leading to a polymer containing monomer units based on formula (Z1d) below: 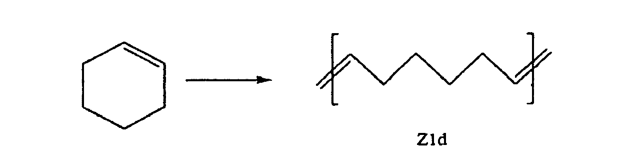
- cyclohexadiene leading to a polymer containing monomer units based on formula (Z1e) below: 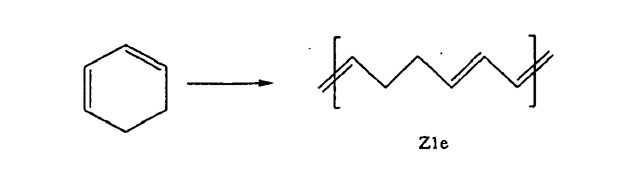
- cycloheptene leading to a polymer containing monomer units based on formula (Z1f) below: 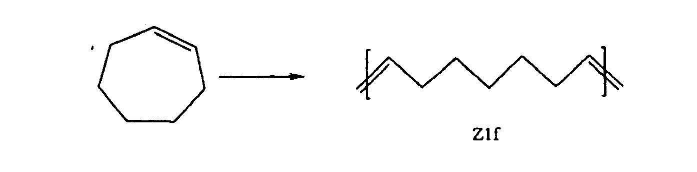
- cyclooctene leading to a polymer containing monomer units based on formula (Z1h) below: 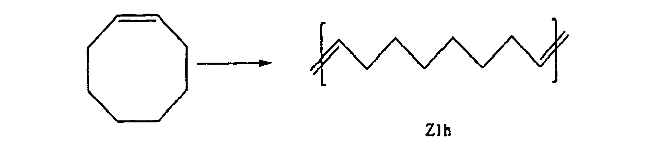
- cyclooctapolyene, in particular cycloocta-1,5-diene, leading to a polymer containing monomer units based on formula (Z1i) below: 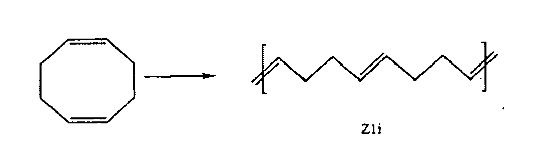
- cyclononene leading to a polymer containing monomer units based on formula (Z1j) below: 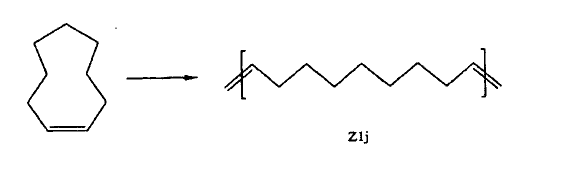
- cyclononadiene leading to a polymer containing monomer units based on formula (Z1k) below: 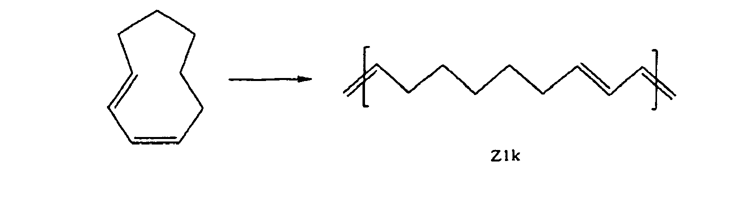
- cyclodecene leading to a polymer containing monomer units based on formula (Z1l) below: 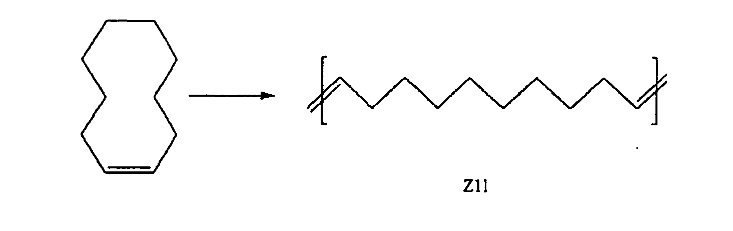
- cyclodeca-1,5-diene leading to a polymer containing monomer units based on formula (Z1m) below: 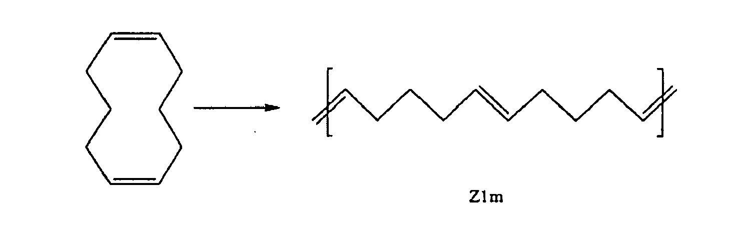
- cyclododecene leading to a polymer containing monomer units based on formula (Z1n) below: 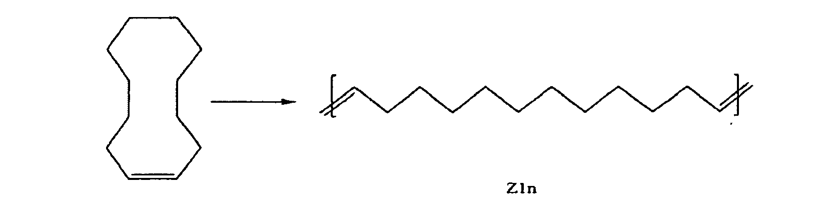
- or alternatively 2,3,4,5-tetrahydrooxepin-2-yl acetate, cyclopentadecene, paracyclophane, ferrocenophane.

4. Spherical particles as claimed in claim 1, **characterised in that** the monomer units are obtained by polymerising polycyclic alkenes and are:
- based on formula (Z2) below
=[CH-R₂-CH]= (Z2)
in which R₂ stands for:
* a cycle based on formula 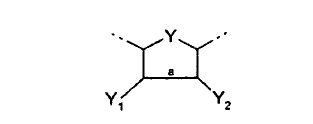 in which:
- Y stands for -CH₂- or a heteroatom or a -CHR- group or a -CHX- group, R and X being as defined in claim 1,
- Y₁ and Y₂, independently of one another, stand for H or an aforementioned chain R or group X, or form a cycle of 4 to 8 carbon atoms in association with the carbon atoms carrying them, this cycle being substituted, depending on the case, by an aforementioned chain R or group X,
- a stands for a single or double bond,
* or a cycle based on formula 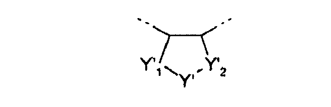 in which:
- Y' stands for -CH₂- or a heteroatom or a -CHR- group or a -CHX- group, R and X being as defined above,
- Y'₁ and Y'₂, independently of one another, stand for -CH₂- or a -C(O) group or a -COR group or a -C-OX group, R and X being as defined above,
- of formula (Z3) below 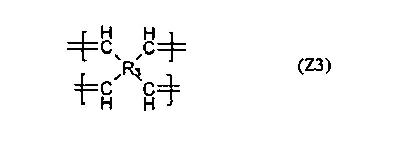 in which R₃ stands for:
* a cycle based on formula 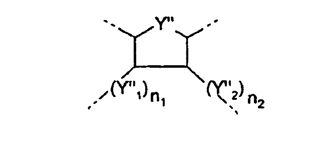 in which:
- n₁ and n₂, independently of one another, stand for 0 or 1,
- Y" stands for -CH₂- or a -CHR- group or a -CHX- group, R and X being as defined above,
- Y"₁ and Y"₂, independently of one another, stand for a hydrocarbonated chain of 0 to 10 carbon atoms,
* or a cycle based on formula 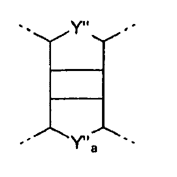 in which Y" and Y"ₐ, independently of one another, stand for -CH₂- or a -CHR- group or a -CHX- group, R and X being as defined above,
* or a cycle based on formula 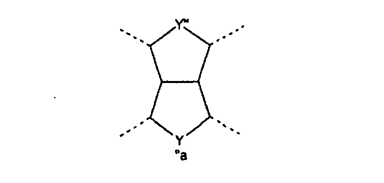
in which Y" and Y"ₐ, independently of one another, stand for -CH₂- or a -CHR- group or a -CHX- group, R and X being as defined above.

5. Spherical particles as claimed in claim 1 or 4, **characterised in that** the polycyclic alkenes from which the monomer units are obtained are:
- the monomers containing a cyclobutene cycle leading to a polymer containing monomer units based on formula (Z2a) below: 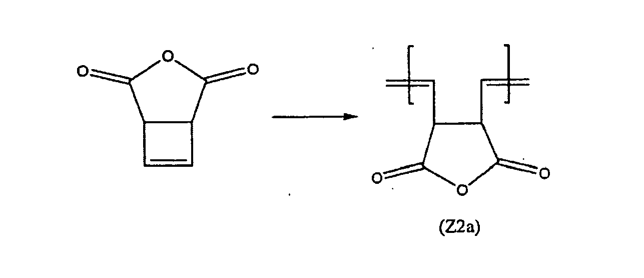
- the monomers containing a cyclopentene cycle leading to a polymer containing monomer units based on formula (Z2b) below: 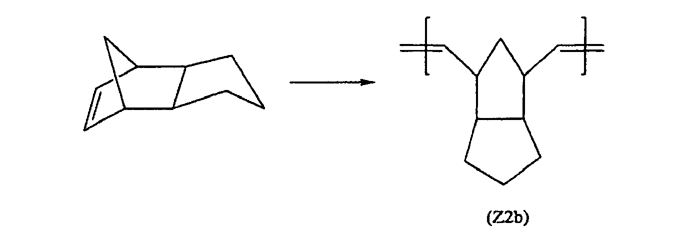
- norbornene (bicyclo[2.2.1]hept-2-ene) leading to a polymer containing monomer units based on formula (Z2c) below: 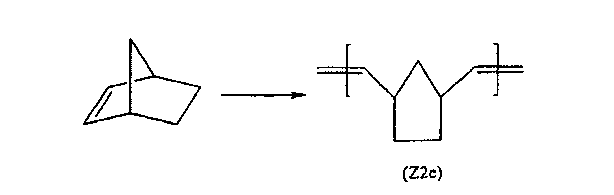
- norbornadiene leading to a polymer containing monomer units based on formula (Z2d) below: 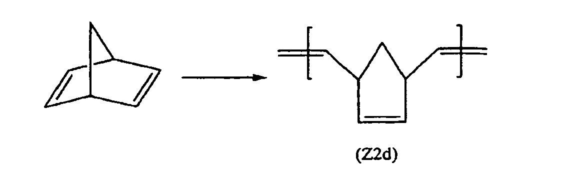
- 7-oxanorbornene leading to a polymer containing monomer units based on formula (Z2e) below: 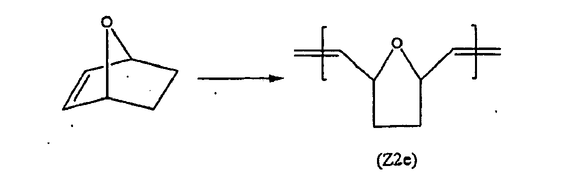
- 7-oxanorbornadiene leading to a polymer containing monomer units based on formula (Z2f) below: 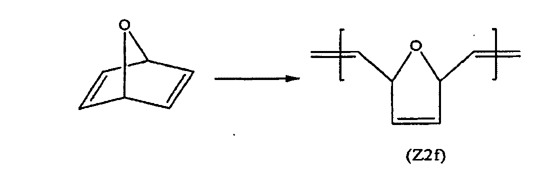
- the dimer of norbornadiene leading to a polymer containing monomer units based on formula (Z3a) below: 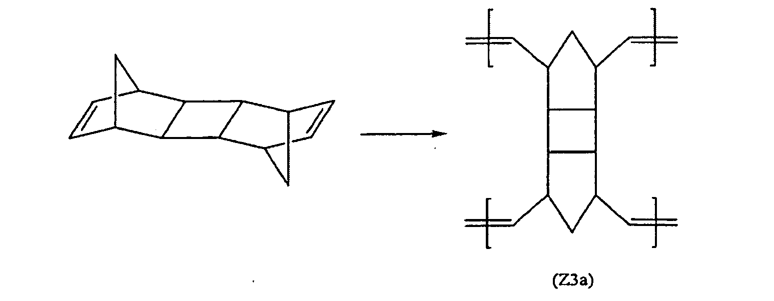
- dicyclopentadiene leading to a polymer containing monomer units based on formula (Z3b) below: 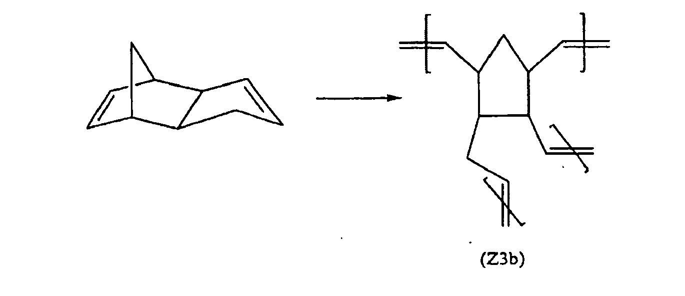
- tetracyclododecadiene leading to a polymer containing monomer units based on formula (Z3c) below: 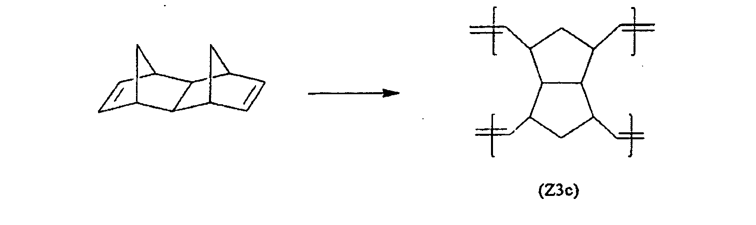
- or bicyclo[5.1.0]oct-2-ene, bicyclo[6.1.0]non-4-ene.

6. Spherical particles as claimed in one of claims 1 to 5, **characterised in that** the mono or polycyclic alkenes from which the monomer units are obtained are:
- norbornene (bicyclo[2.2.1]hept-2-ene) leading to a polymer containing monomer units based on formula (Z2c),
- tetracyclododecadiene leading to a polymer containing monomer units based on formula (Z3c),
- dicyclopentadiene leading to a polymer containing monomer units based on formula (Z3b),
- the dimer of norbornadiene leading to a polymer containing monomer units based on formula (Z3a),
- cycloocta-1,5-diene leading to a polymer containing monomer units based on formula (Z1i).

7. Spherical particles as claimed in one of claims 1 to 6, **characterised in that** at least
0.5%
up to 100% of the monomer units are substituted by a chain R as defined in claim 1.

8. Spherical particles as claimed in one of claims 1 to 7, **characterised in that** they contain:
- between approximately 0.5% to 100% of monomer units substituted by a chain R as defined in claim 1, said chain R being identical for these monomers,
- and between approximately 0.5% and 99.5% of monomer units substituted by a chain R as defined in claim 1, said chain R of these monomers being different from the chain R of the aforementioned monomers,
- and/or between approximately 0.5% and 99.5% of monomer units directly substituted by a group X as defined in claim 1, this group X of these monomers being identical to or different from the group X of the chain R of the aforementioned monomers,
- and/or between approximately 1% and 99.5% of non-substituted monomer units, the total of the percentages of different monomers mentioned above amounting to 100%.

9. Spherical particles as claimed in one of claims 1 to 8, **characterised in that** the chain or chains R substituting the monomer units are represented by the formula
-CH₂-O-(CH₂-CH₂-O)ₙ-CH₂-CH₂-O-X
in which n is as defined in claim 1 and X stands for H, -CH₂-COOH, -CH₂-COCl or - CH₂-COY, Y representing an active ingredient, or a biological molecule such as a protein.

10. Spherical particles as claimed in any one of claims 1 to 9, **characterised in that** the chain or chains R contain an ethylene polyoxide based on formula (A) covalently bonded to a particle via a hydrolysable bridge selected from chain formations of approximately 1 to 10 ε-caprolactone units or -OC(O)-, -C(O)OC(O)-, -C(O)-NH- functions.

11. Spherical particles as claimed in one of claims 1 to 10, **characterised in that** the chain or chains R containing an ethylene polyoxide based on formula (A) covalently bonded to a hydrolysable bridge selected from chain formations of approximately 1 to 10 ε-caprolactone units are represented by the formula
-CH₂-(O-CO-(CH₂)₅)ₜ-O-CO-(CH₂)₅-O-CO-(CH₂)₂-CO-O-(CH₂-CH₂-O)ₙ-(CH₂)₂-O-X
in which t stands for a whole number between 1 and 10 and X stands for H, -CH₂-COOH, -CH₂-COCl or -CH₂-COY, Y representing an active ingredient or a biological molecule such as a protein.

12. Spherical particles as claimed in one of claims 1 to 11, **characterised in that** the active ingredient is selected from the molecules used for therapeutic treatments, cosmetics, perfumery or for surface coatings, such as paints and antifouling compounds.

13. Spherical particles as claimed in one of claims 1 to 12, **characterised in that** the active ingredient is a medicinal product used for therapeutic treatment, selected in particular from those covered by the following categories of treatment: anti-inflammatories, in particular indometacine, anti-cancer drugs, antibiotics, anti-coagulants or antimitotics.

14. Spherical particles as claimed in one of claims 1 to 12, **characterised in that** the biological molecule is selected from proteins likely to bind with an intra-cellular or extracellular biological target, or with antibodies or with any other specific ligand.

15. Spherical particles as claimed in claim 14, **characterised in that** the biological molecule is selected from the following proteins: avidin, albumin, growth factors such as VEGF.

16. Pharmaceutical compositions containing spherical particles as claimed in one of claims I to 15, where the different group or groups X contain a pharmaceutically active ingredient, in association with a pharmaceutically acceptable vehicle, depending on the case, in particular for use in parenteral form.

17. Cosmetic compositions containing spherical particles as claimed in one of claims 1 to 15, where the different group or groups X contain an active ingredient used in cosmetics, in association with an appropriate vehicle, depending on the case, in particular for an application in the form of emulsions or creams.

18. Surface coating compositions containing spherical particles as claimed in one of claims 1 to 15 where the different group or groups X contain an active ingredient used for surface coatings, in association with an appropriate vehicle, depending on the case.

19. Method of preparing spherical particles as defined in one of claims 1 to 15, **characterised in that** it involves a step of polymerising a mono or polycyclic alkene as defined in one of claims 1 to 5 substituted by a chain R as defined in claims 1 and 9 to 11, this polymerisation step optionally being conducted in the presence of:
- one or more mono or polycyclic alkenes as defined in one of claims 1 to 5, which may be identical to or different from the one above, and substituted by a chain R as defined in claim 1 and 9 to 11, said chain R being different from that substituting the aforementioned mono or polycyclic alkene,
- and/or one or more mono or polycyclic alkenes as defined in one of claims 1 to 5, which may be identical to or different from that mentioned above and substituted by a group X as defined in claim 1, this group X being identical to or different from the group X of the chain R of the aforementioned alkenes,
- and/or one or more mono or polycyclic alkenes as defined in one of claims 1 to 5, which may be identical to or different from the aforementioned ones, said alkenes not being substituted,
said polymerisation being conducted under the effect of agitation in the presence of a transition metal complex serving as a reaction initiator in particular selected from those of groups IV or VI or VIII, such as ruthenium, osmium, molybdenum, tungsten, iridium, titanium, in a polar or apolar medium, in particular with the aid of the following ruthenium-based complexes: RuCl₃, RuCl₂(PCy₃)₂CHPh.

20. Mono or polycyclic alkenes, **characterised in that** they are substituted by a chain R or a group X as defined in claim 1, with the proviso that alkene based on the following formula is excluded: 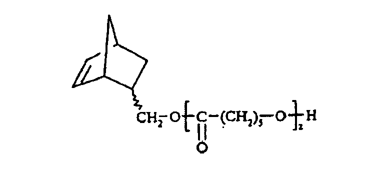 where z stands for a whole number between 1 and 340.

21. Mono or polycyclic alkenes as claimed in claim 20, **characterised in that** they are selected from those specified in claim 3 or 5.

22. Mono or polycyclic alkenes as claimed in claim 20 or 21, **characterised by** the following formulas: 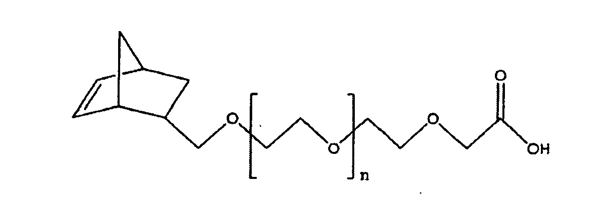 in which n stands for a whole number between approximately 50 and 340, 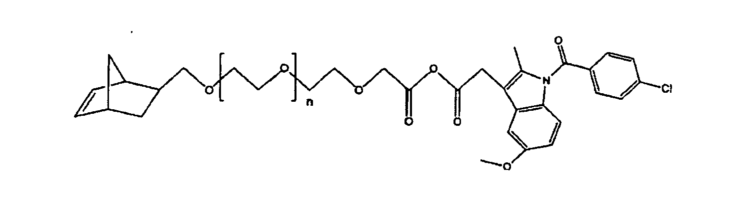 in which n stands for a whole number between approximately 50 and 340, 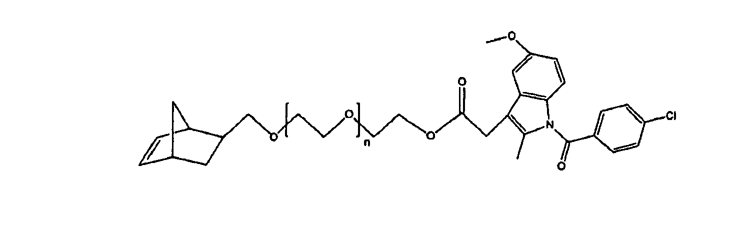 in which n stands for a whole number between approximately 50 and 340, 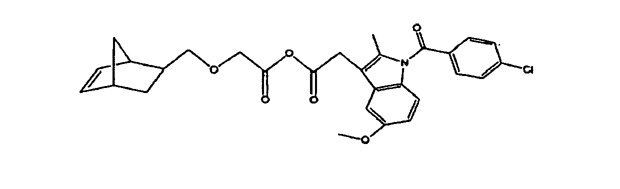 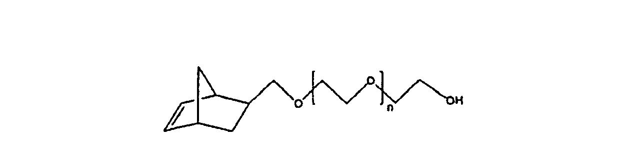 in which n stands for a whole number between approximately 50 and 340, 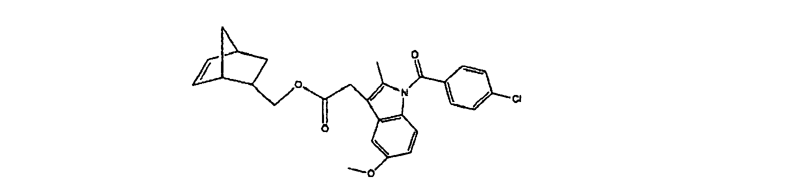 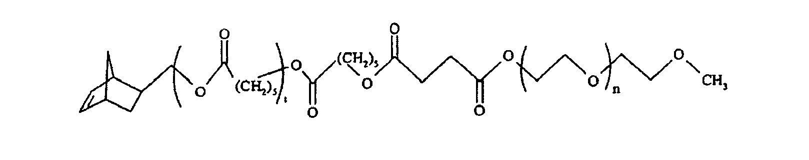 in which t stands for a whole number between 1 and 10 and n stands for a whole number between approximately 50 and 340.

23. Use of mono or polycyclic alkenes as claimed in one of claims 20 to 22 for implementing a method of preparing spherical particles as defined in one of claims 1 to 15, in particular using the method as claimed in claim 19.

## Patentansprüche

1. Kugelförmige Teilchen mit einem Durchmesser zwischen 10 nm und 100 µm, wobei sich die Teilchen aus Polymerketten zusammensetzen, die etwa 30 bis 10000 gleiche oder verschiedene Monomereinheiten enthalten, die aus der Polymerisation monocyclischer Alkene, deren Anzahl den Cyclus bildender Kohlenstoffatome etwa 4 bis 12 ist, oder polycyclischer Alkene, deren Gesamtzahl die Cyclen bildender Kohlenstoffatome etwa 6 bis 20 ist, herrühren, wobei wenigstens eine dieser Monomereinheiten durch eine Kette R substituiert ist, die ein Ethylenpolyoxid der Formel (A) umfasst, das gegebenenfalls über eine hydrolysierbare Brücke kovalent an diese Monomereinheiten gebunden ist
-(CH₂-CH₂-O)ₙ-X (A)
worin n eine ganze Zahl von etwa 50 bis 340, insbesondere 70 bis 200 darstellt und X eine Alkyl- oder Alkyloxykette mit etwa 1 bis 10 Kohlenstoffatomen darstellt, die eine reaktionsfähige funktionelle Gruppe des Typs OH, Halogen, NH₂ oder C(O)X₁ umfasst, worin X₁ ein Wasserstoffatom, Halogenatom, eine Gruppe OR'- oder NHR'- darstellt, worin R' ein Wasserstoffatom oder eine substituierte oder unsubstituierte Kohlenwasserstoffkette mit etwa 1 bis 10 Kohlenstoffatomen darstellt, oder X eine Gruppe darstellt, die eine lichtempfindliche funktionelle Gruppe wie etwa Polyene umfasst, wobei diese lichtempfindliche funktionelle Gruppe gegebenenfalls durch eine Bindung mit einem aktiven Prinzip oder einem biologischen Molekül wie etwa einem Protein gebunden ist und diese Kette R kovalent an die Monomereinheiten gebunden ist.

2. Kugelförmige Teilchen gemäß Anspruch 1, **dadurch gekennzeichnet, dass** die Monomereinheiten aus der Polymerisation monocyclischer Alkene herrühren und von der folgenden Formel (Z1) sind
=[CH-R₁-CH]= (Z1)
worin R₁ eine gesättigte oder ungesättigte Kohlenwasserstoffkette mit 2 bis 10 Kohlenstoffatomen darstellt und die Monomereinheiten gegebenenfalls durch eine in Anspruch 1 definierte Kette R oder direkt durch eine Gruppe X substituiert sind.

3. Kugelförmige Teilchen gemäß Anspruch 1 oder 2, **dadurch gekennzeichnet, dass** die monocyclischen Alkene, aus denen die Monomereinheiten stammen, die folgenden sind:
- Cyclobuten, das zu einem Monomereinheiten der folgenden Formel (Z1a) umfassenden Polymer führt: 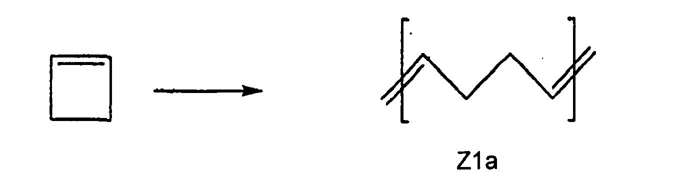
- Cyclopenten, das zu einem Monomereinheiten der folgenden Formel (Z1b) umfassenden Polymer führt: 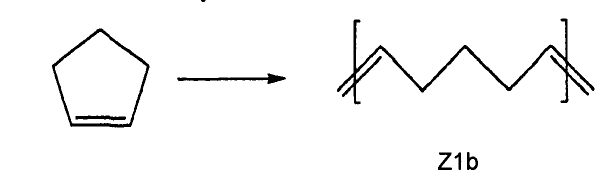
- Cyclopentadien, das zu einem Monomereinheiten der folgenden Formel (Z1c) umfassenden Polymer führt: 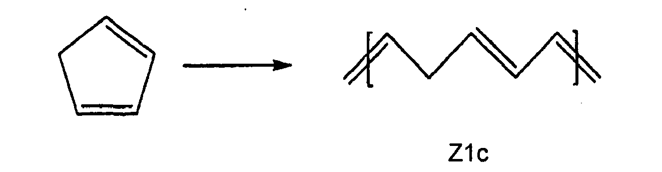
- Cyclohexen, das zu einem Monomereinheiten der folgenden Formel (Z1d) umfassenden Polymer führt: 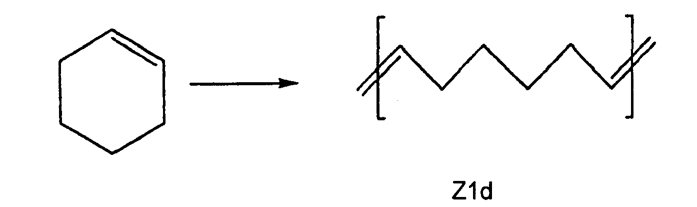
- Cyclohexadien, das zu einem Monomereinheiten der folgenden Formel (Z1e) umfassenden Polymer führt: 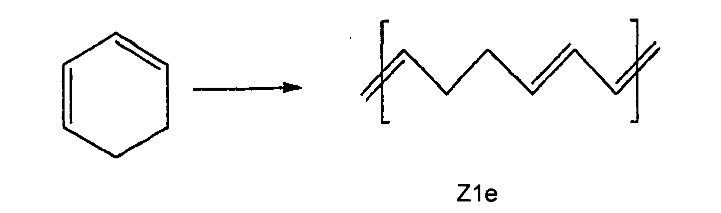
- Cyclohepten, das zu einem Monomereinheiten der folgenden Formel (Z1f) umfassenden Polymer führt: 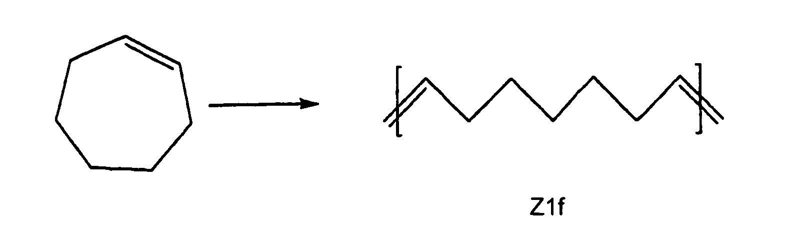
- Cycloocten, das zu einem Monomereinheiten der folgenden Formel (Z1h) umfassenden Polymer führt: 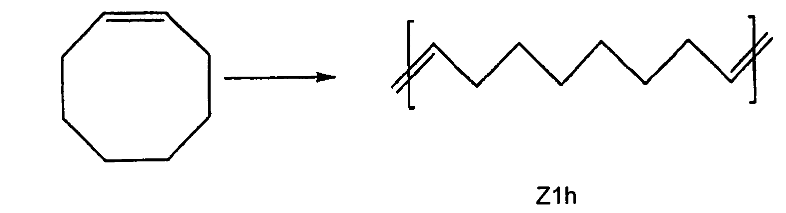
- Cyclooctapolyen, insbesondere Cycloocta-1,5-dien, das zu einem Monomereinheiten der folgenden Formel (Z1i) umfassenden Polymer führt: 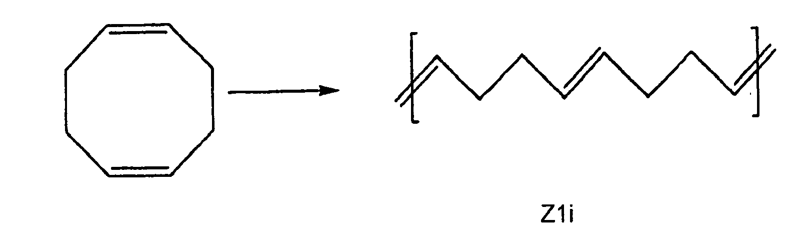
- Cyclononen, das zu einem Monomereinheiten der folgenden Formel (Z1j) umfassenden Polymer führt: 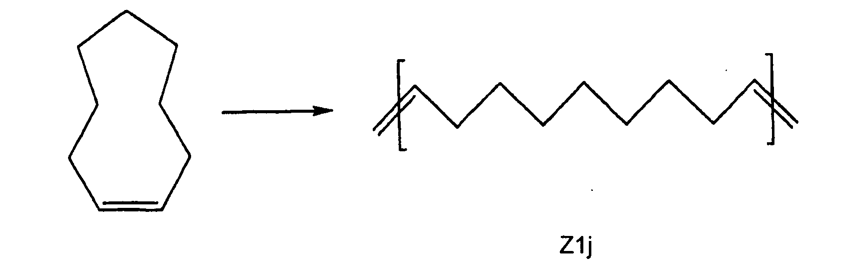
- Cyclononadien, das zu einem Monomereinheiten der folgenden Formel (Z1k) umfassenden Polymer führt: 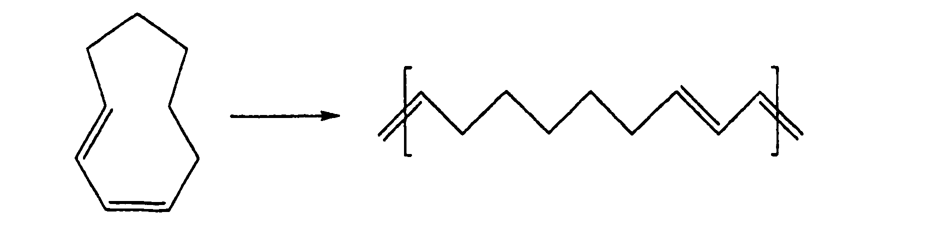
- Cyclodecen, das zu einem Monomereinheiten der folgenden Formel (Z1l) umfassenden Polymer führt: 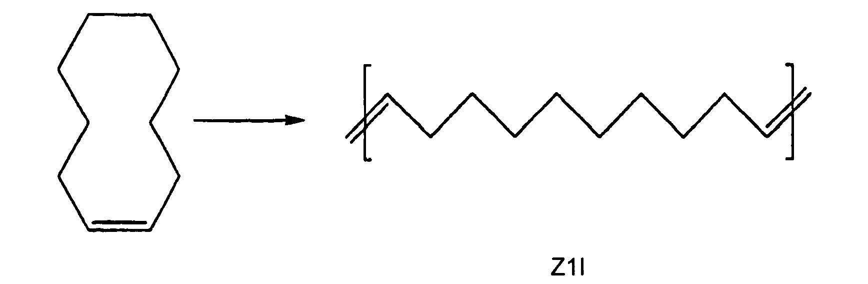
- Cyclodeca-1,5-dien, das zu einem Monomereinheiten der folgenden Formel (Z1m) umfassenden Polymer führt: 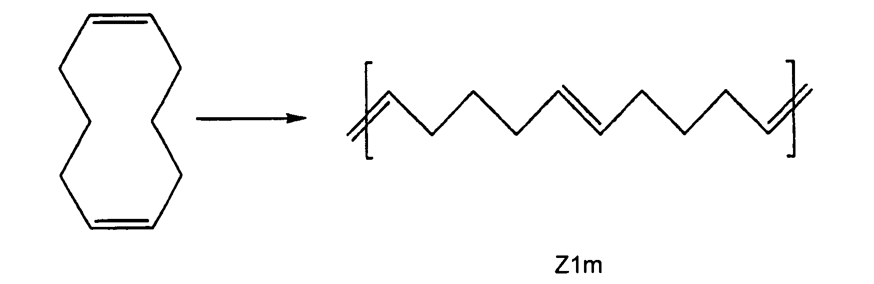
- Cyclododecen, das zu einem Monomereinheiten der folgenden Formel (Z1n) umfassenden Polymer führt: 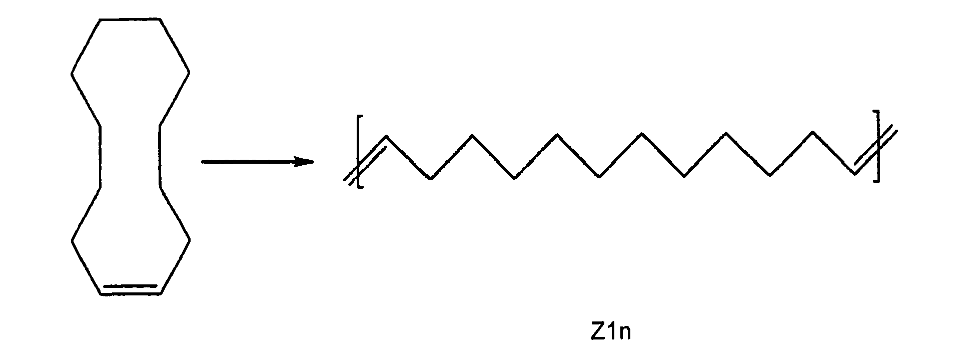
oder auch 2,3,4,5-Tetrahydrooxepin-2-ylacetat, Cyclopentadecen, Paracyclophan und Ferrocenophan.

4. Kugelförmige Teilchen gemäß Anspruch 1, **dadurch gekennzeichnet, dass** die Monomereinheiten aus der Polymerisation polycyclischer Alkene herrühren und von
- der folgenden Formel (Z2)
=[CH-R₂-CH]= (Z2)
worin R₂
* einen Cyclus der Formel 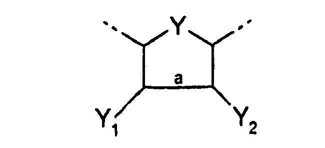 worin
· Y -CH₂- oder ein Heteroatom oder eine Gruppe -CHR- oder eine Gruppe -CHXdarstellt, wobei R und X wie in Anspruch 1 definiert sind,
· Y₁ und Y₂ unabhängig voneinander H oder eine obengenannte Kette R oder Gruppe X darstellen oder zusammen mit den Kohlenstoffatomen, die sie tragen, einen Cyclus mit 4 bis 8 Kohlenstoffatomen bilden, wobei dieser Cyclus gegebenenfalls durch eine obengenannte Gruppe R oder Gruppe X substituiert ist,
· a eine Einfach- oder Doppelbindung darstellt,
* oder einen Cyclus der Formel 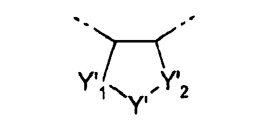 darstellt, worin
· Y'-CH₂- oder ein Heteroatom oder eine Gruppe -CHR- oder eine Gruppe -CHXdarstellt, wobei R und X wie vorstehend definiert sind,
· Y'₁ und Y'₂ unabhängig voneinander -CH₂- oder eine Gruppe -C(O) oder eine Gruppe -COR oder eine Gruppe -C-OX darstellen, wobei R und X wie vorstehend definiert sind, oder
- der folgenden Formel (Z3) sind: 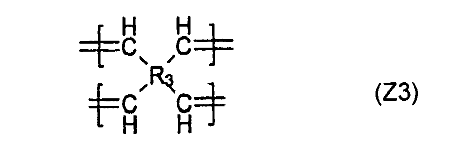 worin R₃
* einen Cyclus der Formel 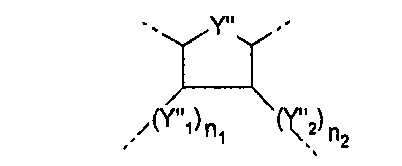 worin
· n₁ und n₂ unabhängig voneinander 0 oder 1 bedeuten,
· Y" -CH₂- oder eine Gruppe -CHR- oder eine Gruppe -CHX- darstellt, wobei R und X wie vorstehend definiert sind,
· Y"₁ und Y"₂ unabhängig voneinander eine Kohlenwasserstoffkette mit 0 bis 10 Kohlenstoffatomen darstellen,
* oder einen Cyclus der Formel 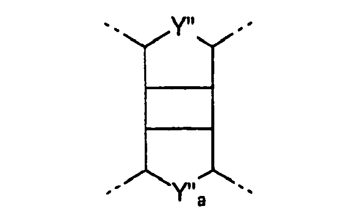 worin Y" und Y"ₐ unabhängig voneinander -CH₂- oder eine Gruppe -CHR- oder eine Gruppe -CHX- darstellen, wobei R und X wie vorstehend definiert sind,
* oder einen Cyclus der Formel 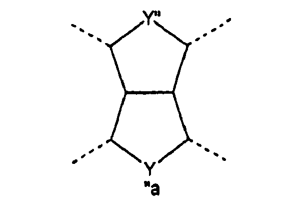 darstellt, worin
Y" und Y"ₐ unabhängig voneinander -CH₂- oder eine Gruppe -CHR- oder eine Gruppe -CHX- darstellen, wobei R und X wie vorstehend definiert sind.

5. Kugelförmige Teilchen gemäß Anspruch 1 oder 4, **dadurch gekennzeichnet, dass** die polycyclischen Alkene, aus denen die Monomereinheiten stammen, die folgenden sind:
- die einen Cyclobutenring enthaltenden Monomeren, die zu einem Monomereinheiten der folgenden Formel (Z2a) umfassenden Polymer führen: 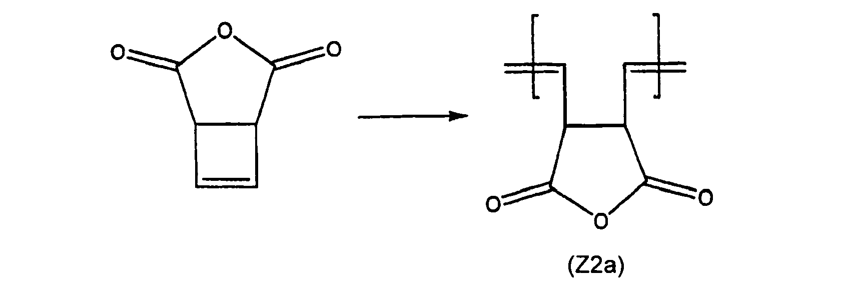
- die einen Cyclopentenring enthaltenden Monomeren, die zu einem Monomereinheiten der folgenden Formel (Z2b) umfassenden Polymer führen: 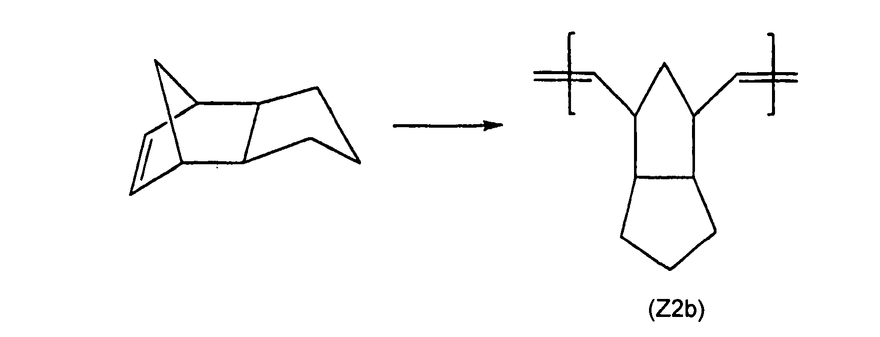
- Norbornen (Bicyclo[2.2.1]hept-2-en), das zu einem Monomereinheiten der folgenden Formel (Z2c) umfassenden Polymer führt: 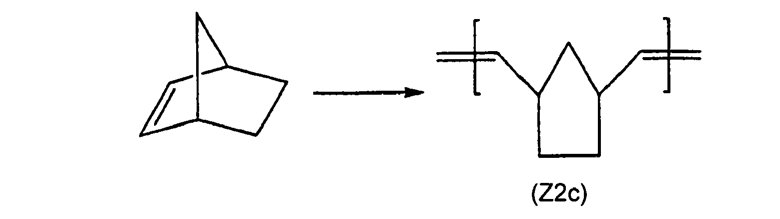
- Norbornadien, das zu einem Monomereinheiten der folgenden Formel (Z2d) umfassenden Polymer führt: 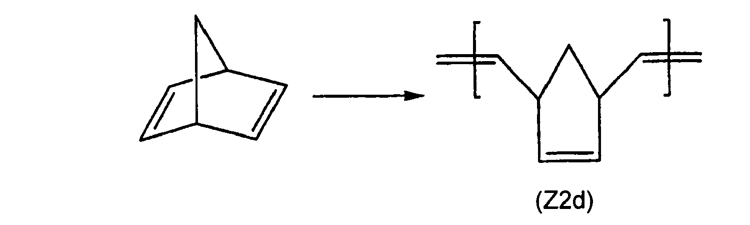
- 7-Oxanorbornen, das zu einem Monomereinheiten der folgenden Formel (Z2e) umfassenden Polymer führt: 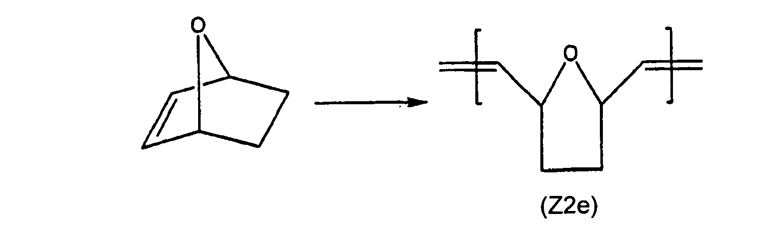
- 7-Oxanorbornadien, das zu einem Monomereinheiten der folgenden Formel (Z2f) umfassenden Polymer führt: 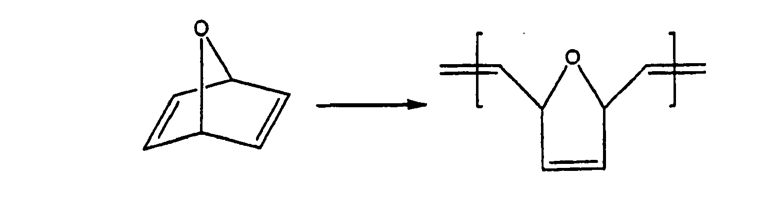 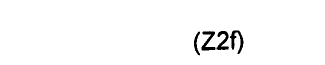
- das Dimer von Norbornadien, das zu einem Monomereinheiten der folgenden Formel (Z3a) umfassenden Polymer führt: 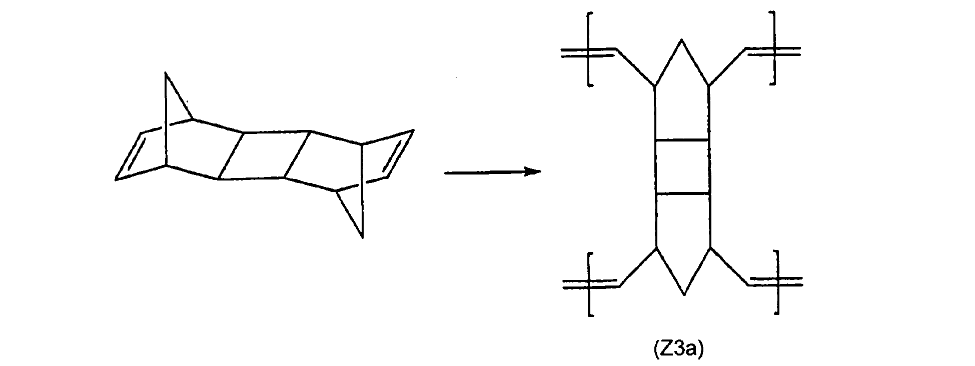
- Dicyclopentadien, das zu einem Monomereinheiten der folgenden Formel (Z3b) umfassenden Polymer führt: 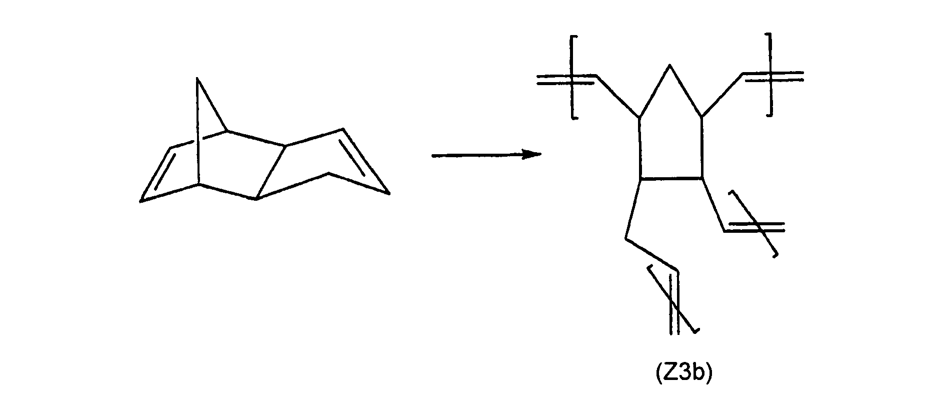
- Tetracyclododecadien, das zu einem Monomereinheiten der folgenden Formel (Z3c) umfassenden Polymer führt: 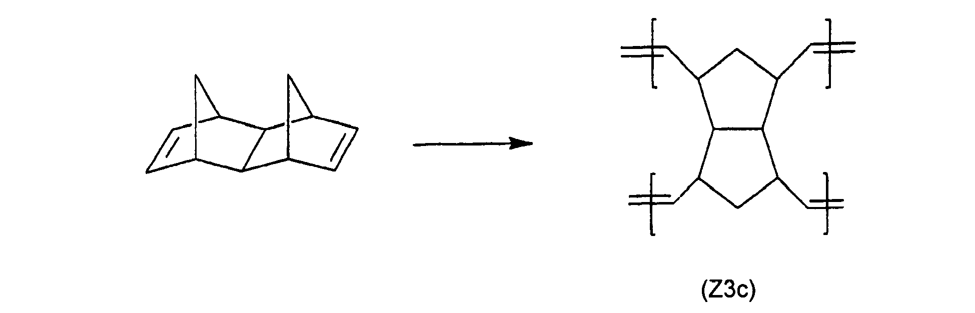
- oder Bicyclo[5.1.0]oct-2-en oder Bicyclo[6.1.0]non-4-en.

6. Kugelförmige Teilchen gemäß einem der Ansprüche 1 bis 5, **dadurch gekennzeichnet, dass** die mono- oder polycyclischen Alkene, aus denen die Monomereinheiten stammen, die folgenden sind:
- Norbornen (Bicyclo[2.2.1]hept-2-en), das zu einem Monomereinheiten der Formel (Z2c) umfassenden Polymer führt,
- Tetracyclododecadien, das zu einem Monomereinheiten der Formel (Z3c) umfassenden Polymer führt,
- Dicyclopentadien, das zu einem Monomereinheiten der Formel (Z3b) umfassenden Polymer führt,
- das Dimer von Norbornadien, das zu einem Monomereinheiten der Formel (Z3a) umfassenden Polymer führt,
- Cycloocta-1,5-dien, das zu einem Monomereinheiten der Formel (Z1i) umfassenden Polymer führt.

7. Kugelförmige Teilchen gemäß einem der Ansprüche 1 bis 6, **dadurch gekennzeichnet, dass** wenigstens 0,5 % bis 100 % der Monomereinheiten durch eine in Anspruch 1 definierte Kette R substituiert sind.

8. Kugelförmige Teilchen gemäß einem der Ansprüche 1 bis 7, **dadurch gekennzeichnet, dass** sie Folgendes umfassen:
- zwischen etwa 0,5 % bis 100 % durch eine in Anspruch 1 definierte Kette R substituierte Monomereinheiten, wobei die Kette R bei diesen Monomeren gleich ist,
- und zwischen etwa 0,5 % bis 99,5 % durch eine in Anspruch 1 definierte Kette R substituierte Monomereinheiten, wobei die Kette R bei diesen Monomeren von der Kette R der voranstehenden Monomeren verschieden ist,
- und/oder zwischen etwa 0,5 % und 99,5 % direkt durch eine in Anspruch 1 definierte Gruppe X substituierte Monomereinheiten, wobei diese Gruppe X dieser Monomeren zu der Gruppe X der Kette R der voranstehenden Monomeren gleich oder verschieden ist,
- und/oder zwischen etwa 1 % und 99,5 % unsubstituierte Monomereinheiten, wobei der gesamte Prozentsatz der vorgenannten verschiedenen Monomeren 100 % beträgt.

9. Kugelförmige Teilchen gemäß einem der Ansprüche 1 bis 8, **dadurch gekennzeichnet, dass** die die Monomereinheiten substituierende Kette oder Ketten R durch die Formel
- CH₂-O-(CH₂-CH₂-O)n-CH₂-CH₂-O-X
dargestellt werden, worin n wie in Anspruch 1 definiert ist und X H, -CH₂-COOH,
- CH₂-COCI oder -CH₂-COY darstellt und Y ein aktives Prinzip oder ein biologisches Molekül wie etwa ein Protein darstellt.

10. Kugelförmige Teilchen gemäß einem der Ansprüche 1 bis 9, **dadurch gekennzeichnet, dass** die Kette oder Ketten R ein Ethylenpolyoxid der Formel (A) umfassen, das über eine aus Ketten von etwa 1 bis 10 ε-Caprolactamstruktureinheiten oder die funktionellen Gruppen -OC(O)-, -C(O)OC(O)- und -C(O)-NH- ausgewählte hydrolysierbare Brücke kovalent an ein Teilchen gebunden ist.

11. Kugelförmige Teilchen gemäß einem der Ansprüche 1 bis 10, **dadurch gekennzeichnet, dass** die Kette oder Ketten R, die ein Ethylenpolyoxid der Formel (A) umfassen, das über eine aus Ketten von etwa 1 bis 10 ε-Caprolactamstruktureinheiten ausgewählte Brücke kovalent gebunden ist, durch die Formel
-CH₂-(O-CO-(CH₂)₅)ₜ-O-CO-(CH₂)₅-O-CO-(CH₂)₂-CO-O-(CH₂-CH₂-O)ₙ-(CH₂)₂-O-X
dargestellt werden, worin t eine ganze Zahl zwischen 1 und 10 darstellt und X H, -CH₂-COOH, -CH₂-COCl oder -CH₂-COY darstellt und Y ein aktives Prinzip oder ein biologisches Molekül wie etwa ein Protein darstellt.

12. Kugelförmige Teilchen gemäß einem der Ansprüche 1 bis 11, **dadurch gekennzeichnet, dass** das aktive Prinzip aus therapeutisch, kosmetisch, als Duftstoff oder zu Oberflächenüberzügen wie etwa Anstrichmitteln und Bewuchsverhinderungsmitteln verwendeten Molekülen ausgewählt ist.

13. Kugelförmige Teilchen gemäß einem der Ansprüche 1 bis 12, **dadurch gekennzeichnet, dass** das aktive Prinzip ein therapeutisch verwendetes Arzneimittel ist, das insbesondere aus den folgenden therapeutischen Klassen ausgewählt ist: Entzündungshemmer, insbesondere Indometacin, Antikrebsmitteln, Antibiotika, Antikoagulantien oder Mitosehemmer.

14. Kugelförmige Teilchen gemäß einem der Ansprüche 1 bis 12, **dadurch gekennzeichnet, dass** das biologische Molekül aus den Proteinen ausgewählt ist, die an ein intra- oder extrazelluläres biologisches Ziel oder an Antikörper oder an irgendeinen anderen spezifischen Liganden binden können.

15. Kugelförmige Teilchen gemäß Anspruch 14, **dadurch gekennzeichnet, dass** das biologische Molekül aus den folgenden Proteinen ausgewählt ist: Avidin, Albumin, Wachstumsfaktoren wie etwa VEGF.

16. Pharmazeutische Zusammensetzungen umfassend kugelförmige Teilchen gemäß einem der Ansprüche 1 bis 15, wobei die verschiedene Gruppe oder Gruppen X ein aktives Arzneimittelprinzip gegebenenfalls in Verbindung mit einem pharmazeutisch annehmbaren Träger, insbesondere zur Verwendung in parenteraler Form enthalten.

17. Kosmetische Zusammensetzung umfassend kugelförmige Teilchen gemäß einem der Ansprüche 1 bis 15, wobei die verschiedene Gruppe oder Gruppen X ein in der Kosmetik verwendetes aktives Prinzip gegebenenfalls in Verbindung mit einem geeigneten Träger, insbesondere zur Anwendung in Form von Emulsionen und Cremes enthalten.

18. Oberflächenüberzugszusammensetzung umfassend kugelförmige Teilchen gemäß einem der Ansprüche 1 bis 15, wobei die verschiedene Gruppe oder Gruppen X ein zu Oberflächenüberzügen verwendetes aktives Prinzip gegebenenfalls in Verbindung mit einem geeigneten Träger enthalten.

19. Verfahren zur Herstellung in einem der Ansprüche 1 bis 15 definierter kugelförmiger Teilchen, **dadurch gekennzeichnet, dass** es einen Schritt der Polymerisation eines in einem der Ansprüche 1 bis 5 definierten, durch eine in den Ansprüchen 1 bis 9 und 11 definierte Kette R substituierten mono- oder polycyclischen Alkens umfasst, wobei dieser Polymerisationsschritt gegebenenfalls in Anwesenheit
- eines oder mehrerer, in einem der Ansprüche 1 bis 5 definierter, mono- oder polycyclischer, zum voranstehenden gleicher oder verschiedener und durch eine in den Ansprüchen 1 bis 9 und 11 definierte Kette R substituierter Alkene, wobei diese Kette R von der das vorgenannte mono- oder polycyclische Alken substituierenden verschieden ist,
- und/oder eines oder mehrerer, in einem der Ansprüche 1 bis 5 definierter, mono- oder polycyclischer, zum voranstehenden gleicher oder verschiedener und durch eine in Anspruch 1 definierte Gruppe X substituierter Alkene, wobei diese Gruppe X zu der Gruppe X der Kette R der voranstehenden Alkene gleich oder verschieden ist,
- und/oder eines oder mehrerer, in einem der Ansprüche 1 bis 5 definierter, zu den voranstehenden gleicher oder verschiedener, mono- oder polycyclischer Alkene, die unsubstituiert sind, ausgeführt wird,
wobei die Polymerisation unter Rühren in Gegenwart eines Komplexes insbesondere eines aus den Gruppen IV oder VI oder VIII ausgewählten Übergangsmetalls wie etwa Ruthenium, Osmium, Molybdän, Wolfram, Iridium und Titan als Initiator der Reaktion in polarem oder apolarem Medium, insbesondere mit Hilfe der folgenden Komplexe auf Rutheniumgrundlage ausgeführt wird: RuCl₃, RuCl₂(PCy₃)₂CHPh.

20. Mono- oder polycyclische Alkene, **dadurch gekennzeichnet, dass** sie durch eine in Anspruch 1 definierte Kette R oder eine Gruppe X substituiert sind, mit der Maßgabe, dass das Alken der folgenden Formel ausgeschlossen ist: 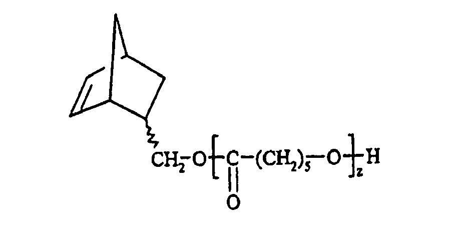 worin z eine ganze Zahl zwischen 1 und 340 darstellt.

21. Mono- oder polycyclische Alkene gemäß Anspruch 20, **dadurch gekennzeichnet, dass** sie aus den in Anspruch 3 oder 5 angeführten ausgewählt sind.

22. Mono- oder polycyclische Alkene gemäß Anspruch 20 oder 21, die durch die folgenden Formeln **gekennzeichnet** sind: 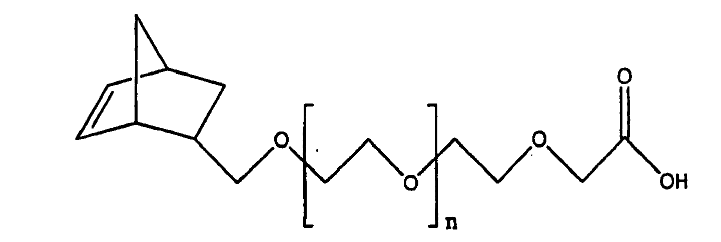 worin n eine ganze Zahl zwischen etwa 50 und 340 darstellt, 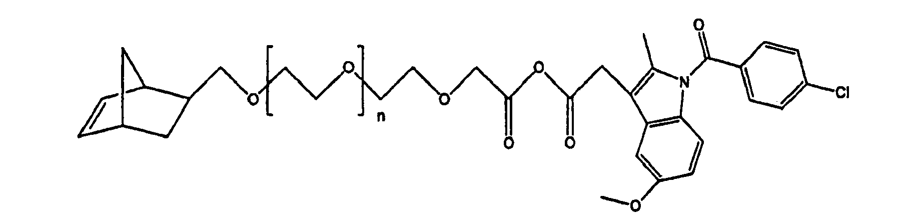 worin n eine ganze Zahl zwischen etwa 50 und 340 darstellt, 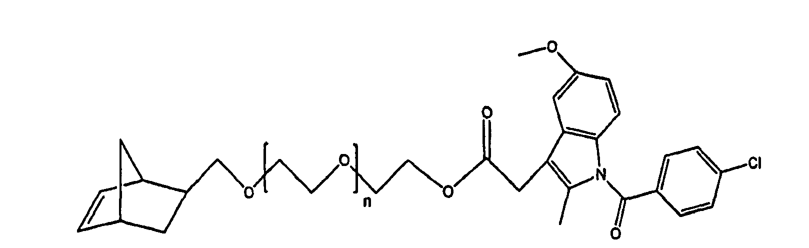 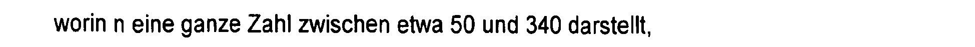 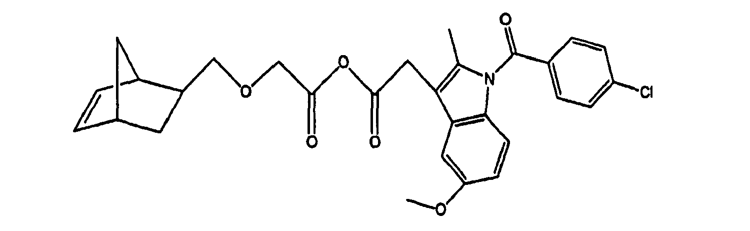 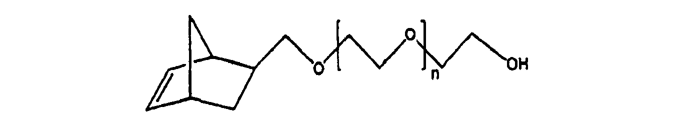 worin n eine ganze Zahl zwischen etwa 50 und 340 darstellt, 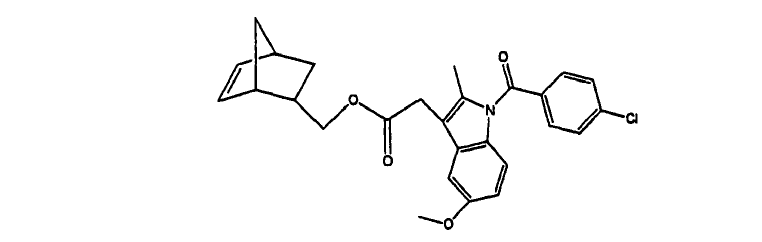 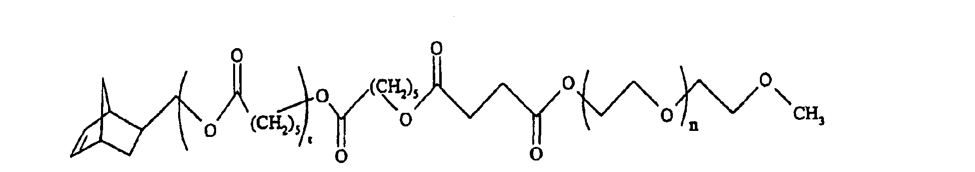 worin t eine ganze Zahl zwischen 1 und 10 darstellt und n eine ganze Zahl zwischen etwa 50 und 340 darstellt.

23. Verwendung mono- oder polycyclischer Alkene gemäß einem der Ansprüche 20 bis 22 zum Einsatz in einem Verfahren zur Herstellung in einem der Ansprüche 1 bis 15 definierter kugelförmiger Teilchen, insbesondere gemäß dem Verfahren des Anspruchs 19.
